# EUROPEAN PATENT SPECIFICATION

(11) **EP 0 876 398 B1**
(45) Date of publication and mention of the grant of the patent: **17.07.2002**
(21) Application number: 97902074.0
(22) Date of filing: 23.01.1997
(51) Int. Cl.: C07K 7/08, C07K 9/00, A61K 39/00

(54) **INDUCTION OF IMMUNE RESPONSE AGAINST DESIRED DETERMINANTS**
DIE ERZEUGUNG EINER IMMUNANTWORT GEGEN ERWÜNSCHTE DETERMINANTEN
INDUCTION D'UNE REACTION IMMUNE CONTRE DES DETERMINANTS SOUHAITES

(30) Priority: 24.01.1996 US 10510 P
(43) Date of publication of application: 11.11.1998
(73) Proprietor: Epimmune, Inc., San Diego, California 92121 (US)
(72) Inventor: ALEXANDER, Jeffery, L., San Diego, CA 92130 (US); DEFREES, Shawn, San Marcos, CA 92069 (US); SETTE, Alessandro, La Jolla, CA 92037 (US)
(74) Representative: Bowman, Paul Alan
(86) International application number: US9701041
(87) International publication number: WO9726784

(56) References cited:
- WO-A-94/20127
- WO-A-95/07707
- IMMUNITY, vol. 1, no. 9, December 1994, pages 751-761, XP000673954 J. ALEXANDER ET AL.: "Development of High Potency Universal DR-Restricted Helper Epitopes by Modification of High Affinity DR-Blocking Peptides" cited in the application
- JOURNAL OF IMMUNOLOGY, vol. 148, no. 8, 15 April 1992, BALTIMORE US, pages 2446-2451, XP002032422 G.Y. ISHIOKA ET AL.: "MHC Interaction and T Cell Recognition of Carbohydrates and Glycopeptides"
- SPRINGER SEMINARS IN IMMUNOPATHOLOGY, vol. 15, no. 2/3, 1993, pages 293-302, XP000674368 G.Y. ISHIOKA ET AL.: "Major histocompatibility complex class II associated and induction of T cell responses by carbohydrates and glycopeptides"
- VACCINE, vol. 12, no. 10, August 1994, GUILDFORD GB, pages 867-871, XP002032423 S. HERV S-STUBBS ET AL.: "Overcoming the class II-linked non-responsiveness to hepatits B vaccine" cited in the application
- VACCINE, vol. 13, no. 5, 1995, GUILDFORD GB, pages 463-470, XP002032424 A. BARTOLONI ET AL.: "Immunogenicitiy of meningococcal B polysaccharide conjugated to tetanus toxoid or CRM197 via adipic acid dihydrazide" cited in the application
- INFECTION AND IMMUNITY, vol. 40, no. 1, April 1983, WASHINGTON US, pages 245-256, XP002032425 C. CHU ET AL.: "Further Studies on the Immunogenicity of Haemophilus influenzae Type b and Pneumococcal Type 6A Polysaccharide-Protein Conjugates" cited in the application

## Description

### BACKGROUND OF THE INVENTION

It is generally known in the art that the nature of the immune response raised against a particular vaccine antigen is important to the overall effectiveness of the vaccine. In the case of carbohydrate antigens, a large variety of approaches has been explored in attempts to enhance their immunogenicity, including chemical modification (Jennings, *et al*. in *Towards Better Carbohydrate Vaccines* Bell and Torrigiani (eds) pp 11-17, J. Wiley & Sons, London, 1987), administration with adjuvants, noncovalent complexing with proteins, covalent attachment to immunogenic protein carriers (Schneerson, *et al.* in *Towards Better Carbohydrate Vaccines*, *supra* pp 307-327), and replacement of the carbohydrate epitope by a protein replica, either peptides synthesized *de novo* (so-called mimitopes, Geyson, *et al.* in *Towards Better Carbohydrate Vaccines*, *supra*, pp 103-118) or antiidiotypic antibodies (Soederstroem in *Towards Better Carbohydrate Vaccines*, *supra*, pp 119-138).

Covalent attachment of carbohydrate antigens to immunogenic T-dependent protein carriers is known *(see, e.g.,* Schneerson, *et al*., **152**:361-376 (1980); Lepow, *et al*., *J. Pediatr.* **106**:185-189 (1985); Chu, *et al*., *Infect. Immun*., **50**:245-256 (1983); Marborg *et al*., Am. Chem. Soc.) 108:5282-5287 (1985); Anderson *et al*., *Infect. Immun*., **39**:233-238 (1983); Bartoloni, *et al*., *Vaccine* **13**:463-470 (1995); and Wessels, *et al., J. Infect. Dis.* **171**:879-884 (1995)).

Immunogenic peptides, containing epitopes recognized by T helper cells, have been found to be useful in inducing immune responses. The use of helper peptides to enhance antibody responses against particular determinants is described for instance in Hervas-Stubbs, *et al*., *Vaccine* **12**:867-871 (1994).

Although allele-specific polymorphic residues that line the peptide binding pockets of MHC alleles tend to endow each allele with the capacity to bind a unique set of peptides, there are many instances in which a given peptide has been shown to bind to more than one MHC allele. This has been best documented in the case of the human DR isotype, in which it has been noted that several DR alleles appear to recognize similar motifs, and independently, several investigators reported degenerate binding and/or recognition of certain epitopes in the context of multiple DR types, leading to the concept that certain peptides might represent "universal" epitopes (Busch, *et al*., *Int. Immunol.* **2**:443-451 (1990); Panina-Bordignon, *et al*., *Eur. J. Immunol.* **19**:2237-2242 (1989); Sinigaglia, *et al*., *Nature* **336**:778-780 (1988); O'Sullivan, *et al*., *J. Immunol.* **147**:2663-2669 (1991); Roache, *et al*., *J. Immunol.* **144**:1849-1856 (1991); Hill, *et al*., *J. Immunol.* **147**:189-197 (1991)). Although, the previously reported peptides do have the capacity to bind to several DR alleles, they are by no means universal.

Pan-DR binding (PADRE) peptides have been described in WO 95/07707 and Alexander, *et al.*, *Immunity* **1**:751-761 (1994). These peptides have been shown to help in the generation of a CTL response against desired protein antigens.

Springer Seminor Immunopathology, vol. 15 (1993), pages 293-302 discusses major histocompatibility complex class II association and induction of T cell responses by carbohydrates and glycopeptides.

However, the prior art fails to teach compounds or compositions that provide an effective humoral response. For example, in the case of carbohydrate immunogens, antibody responses have traditionally been predominantly of short-term expression of IgM followed by an inconsistent IgG response. Such a response is generally not as effective in producing long term protection against the immunogen as an IgG-mediated response. The present invention addresses these and other needs.

### BRIEF SUMMARY OF THE INVENTION

The present invention provides compositions comprising a PADRE oligopeptide of less than about 50 amino acid residues and an antigenic determinant, wherein the oligopeptide and antigenic determinant are optionally covalently attached to each other. The antigenic determinant can be from a bacterium, a virus, a cancer cell, a fungus, or a parasite. When the PADRE oligopeptide and the antigenic determinant are covalently attached to each other, they will be either directly linked or attached by means of a linking group which preferably comprises a cysteine residue.

In one group of embodiments, the PADRE peptide is selected from the group consisting of aAXAAAKTAAAAa, aAXAAAATLKAAa, aAXVAAATLKAAa, aAXIAAATLKAAa, aKXVAAWTLKAAa, and aKFVAAWTLKAAa wherein a is D-alanine, A is L-alanine, X is cyclohexylalanine, K is lysine, T is threonine, L is leucine, V is valine, I is isoleucine, W is tryptophan, and F is phenylalanine. More preferably, the PADRE peptide is aKXVAAWTLKAAa. In other groups of embodiments, the termini of the peptides can be either in the D- or L- form.

Additionally, the present invention provides a composition for eliciting an immune response to an immunogenic carbohydrate, the composition comprising a PADRE oligopeptide of less than about 50 residues and at least one carbohydrate epitope. Preferably, the PADRE peptide has the formula R₁-R₂-R₃-R₄-R₅, proceeding from the N-terminus to the C-terminus, wherein R₁ consists of at least 2 residues; R₂ is selected from the group consisting of a cyclohexylalanine residue, a tyrosine residue, a phenylalanine residue and conservative substitutions therefor; R₃ is 3 to 5 amino acid residues; R₄ is selected from the group consisting of threonine-leucine-lysine, lysine-threonine, and tryptophan-threonine-leucine-lysine, and conservative substitutions therefor; and R₅ consists of at least 2 residues.

### BRIEF DESCRIPTION OF THE FIGURES

Figures 1A-1F show representative responses for three of twelve different donors. Antigen specific T cell responses from human PBMC T cell lines generated on day 0 by addition of either peptide 965.10 (closed square), 906.09 (open square), 760.50 (closed circle), or Tetanus toxoid 830-843 (open circle) as assayed on day 14 (second stimulation, Figures 1A, 1B, 1C) and day 28 (third stimulation, Figures 1D, 1E, 1F). A representative of two independent experiments is shown.
Figures 2A, 2B show summaries of antigen specific T cell responses from human PBMC. Figure 2A shows results of a second stimulation, and Figure 2B shows results of a third stimulation. The maximum Δ cpm obtained is plotted on the ordinate.
Figures 3A, 3B show summaries of antigen specific T cell responses from human PBMC. Figure 3A shows results of a second stimulation, and Figure 3B shows results of a third stimulation. The maximum Δ cpm obtained is plotted on the ordinate.

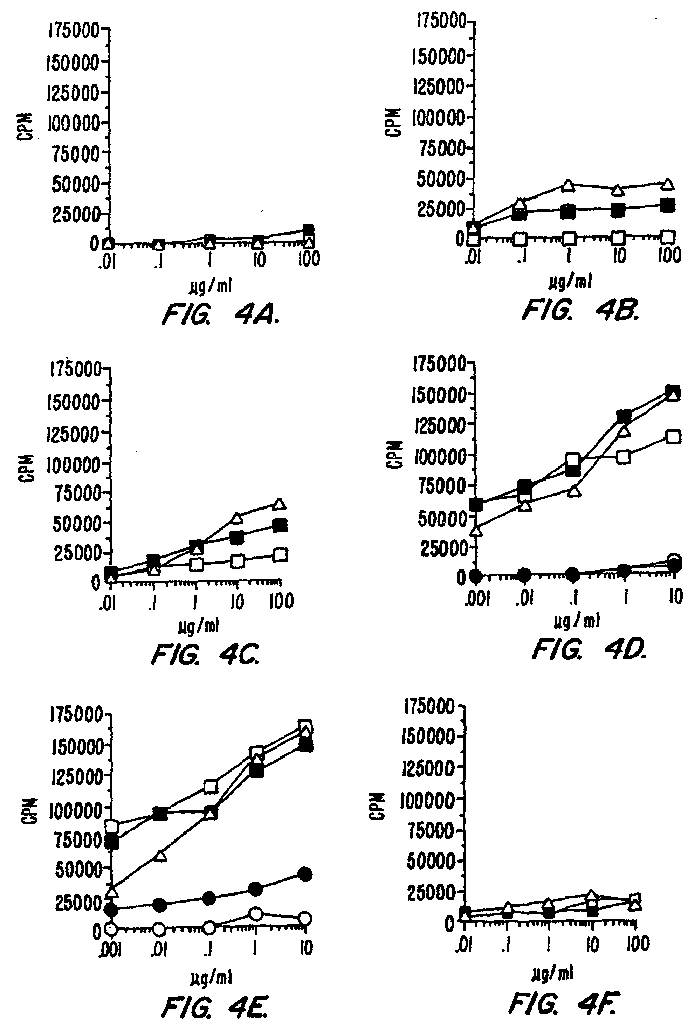

Figures 4A-4F show *in vivo* immunogenicity of various peptide epitopes as measured by proliferative capacity of primed murine lymph node T cells. C57BL/6J mice were injected with 20 µg/mouse (open triangle), 1µg/mouse (closed square), 50 ng/mouse (open square), 2.5 ng/mouse (closed circle), or 0.125 ng/mouse (open circle) of TT 830-843 (Figure 4A), Ova 323-336 (Figure 4B), HBV_{c} 128-140 (Figure 4C), 965.10 (Figure 4D) 1024.03 (Figure 4E), and 760.50 (Figure 4F). Ten days later, draining lymph nodes were removed and T cell proliferation assays performed as described in detail below. A representative of two independent experiments is shown.
Figures 5A, B and C indicate the structures of the pentasaccharide fucopentaose II and its conjugates; (A) fucopentaose II-PADRE, (B) fucopentaose II-PA14, and (C) fucopentaose II-HSA.
Figures 6A and B represent the structures of (A) dodecasaccharide-PADRE conjugate and (B) *Salmonella typhimurium* LPS.

### DETAILED DESCRIPTION OF THE INVENTION

### A. Definitions

An oligopeptide or peptide as used herein refers to a chain of at least four amino acid or amino acid mimetics, preferably at least six, more preferably eight to ten, sometimes eleven to fourteen residues, and usually fewer than about fifty residues, more usually fewer than about twenty-five, and preferably fewer than fifteen, e.g., eight to fourteen residues. The oligopeptides or peptides can be a variety of lengths, either in their neutral (uncharged) forms or in forms which are salts, and either free of modifications such as glycosylation, side chain oxidation, or phosphorylation or containing these modifications, subject to the condition that the modification not destroy the biological activity of the polypeptides as herein described.

When referring to an amino acid residue in a peptide, oligopeptide or protein the terms "amino acid residue", "amino acid" and "residue" are used interchangeably and, as used herein, mean an amino acid or amino acid mimetic joined covalently to at least one other amino acid or amino acid mimetic through an amide bond or amide bond mimetic.

As used herein, the term "amino acid", when unqualified, refers to an "L-amino acid" or L-amino acid mimetic.

Although the peptides will preferably be substantially free of other naturally occurring proteins and fragments thereof, in some embodiments the peptides can be synthetically conjugated to native fragments or particles.

As used herein, the term "biological activity" means the ability to bind an appropriate MHC molecule and, in the case of peptides useful for stimulating immune responses, induce a T helper response, which in turn helps to induce an immune response against a target immunogen or immunogen mimetic. In the case of peptides useful for stimulating antibody responses, the peptide will induce a T helper response, which in turn helps induce a humoral response against the target immunogen.

A "pan DR-binding peptide (PADRE)" of the invention is a peptide capable of binding at least about 7 of the 12 most common DR alleles (DR1, 2w2b, 2w2a, 3, 4w4, 4w14, 5, 7, 52a, 52b, 52c, and 53).

Throughout this disclosure, results are expressed in terms of IC50s. Given the conditions in which the assays are run (i.e., limiting MHC and labeled peptide concentrations), these values approximate K_{D} values. It should be noted that IC50 values can change, often dramatically, if the assay conditions are varied, and depending on the particular reagents used (*e.g.*, MHC preparation, etc.). For example, excessive concentrations of MHC will increase the apparent measured IC50 of a given ligand.

An alternative way of expressing the binding data, to avoid these uncertainties, is as a relative value to a reference peptide. The reference peptide is included in every assay. As a particular assay becomes more or less sensitive, the IC50 of the peptides tested may change somewhat. However, the binding relative to the reference peptide will not change. For example, in an assay run under conditions such that the IC50 of the reference peptide increases 10-fold, all IC50 values will also shift approximately 10-fold. Therefore, to avoid ambiguities, the assessment of whether a peptide is a good, intermediate, weak, or negative binder should be based on its IC50, relative to the IC50 of the standard peptide.

If the IC50 of the standard peptide measured in a particular assay is different from that reported in Table 1, then it should be understood that the threshold values used to determine good, intermediate, weak, and negative binders should be modified by a corresponding factor.

The PADRE peptides of the invention, in addition to promoting an immune response against a second determinant, can also serve as target immunogens, themselves. Thus, for instance, in the case in which the PADRE peptide is linked to a carbohydrate epitope, the immune response may be to both the PADRE peptide and the carbohydrate epitope.

The terms "immunogen" and "antigen" are used interchangeably and mean any compound to which a cellular or humoral immune response is to be directed against.

As used herein, the term "antigenic determinant" is any structure that can elicit, facilitate, or be induced to produce an immune response, for example carbohydrate epitopes, lipids, proteins, peptides, or combinations thereof.

A "CTL epitope" of the present invention is one derived from selected epitopic regions of potential target antigens, such as tumor associated antigens, including, but not limited to, renal cell carcinoma, breast cancer, carcinoembryonic antigens, melanoma (MAGE-1) antigens, and prostate cancer specific antigen, hepatitis C antigens, Epstein-Barr virus antigens, HIV-1 and HIV-2 antigens, and papilloma virus antigens.

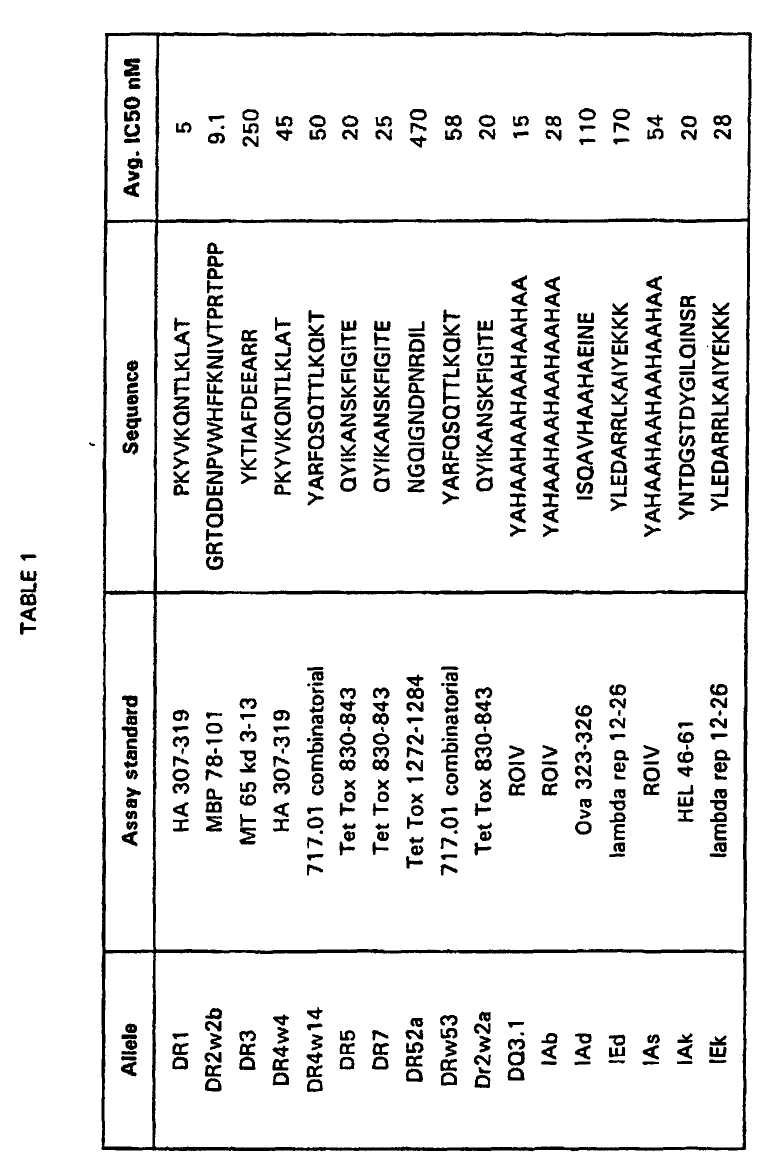

A "humoral response" of the present invention is an antibody-mediated immune response directed towards various regions of an antigenic determinant. One of skill will recognize that a humoral response may also be induced against a PADRE peptide, wherein the PADRE peptide would also be included with the determinant. Thus the elicited immune response may be against both the antibody inducing determinant and the PADRE peptide.

A "carbohydrate epitope" as used herein refers to a carbohydrate structure, present as a glycoconjugate, e.g., glycoprotein, glycopeptide, glycolipid, and the like, DNA, RNA, or a polysaccharide, oligosaccharide, or monosaccharide against which an immune response is desired. The carbohydrate epitope may induce a wide range of immune responses. One of skill will recognize that various carbohydrate structures exemplified herein can be variously modified according to standard methods, without adversely affecting antigenicity. For instance, the monosaccharide units of the saccharide may be variously substituted or even replaced with small organic molecules, which serve as mimetics for the monosaccharide.

The phrases "isolated" or biologically pure" refer to material which is substantially or essentially free from components which normally accompany it as found in its native state. Thus, the peptides of the present invention do not contain materials normally associated with their *in situ* environment, e.g., MHC Class I molecules with antigen presenting cells. Even if a protein has been isolated to a homogeneous or dominant band in an electrophoretic gel, there are trace contaminants in the range of 5-10% of native protein which co-purify with the desired protein. Isolated peptides of this invention do not contain such endogenous co-purified protein.

A "linker" as used herein is any compound used to provide covalent linkage and spacing between two functional groups (e.g., a PADRE peptide and a desired immunogen). Typically, the linker comprises neutral molecules, such as aliphatic carbon chains, amino acids or amino acid mimetics, which are substantially uncharged under physiological conditions and may have linear or branched side chains. In some cases, the linker may, itself, be immunogenic, although non-therapeutically directed. Various linkers useful in the invention are described in more detail, below. Additionally, the verbs "link" and "conjugate" are used interchangeably herein and refer to covalent attachment of two or more species.

The term "T cell clone" refers to a group of T cells that are progeny of a single virgin lymphocyte and express identical T cell receptor proteins. The term "virgin" lymphocyte is used here as it is used in Stites *et al. Basic and Clinical Immunology, 8th Edition,* Prentice Hall, Englewood Cliffs, New Jersey (1994).

A "T helper peptide" as used herein refers to a peptide recognized by the T cell receptor of T helper cells. The PADRE peptides of the present invention are T helper peptides.

### B. PADRE Peptides and Antigenic Determinants

The compositions of the invention generally comprise two components, a PADRE peptide conjugated to or admixed with one or more antigenic determinants. The embodiments in which the two components are linked will have the following general structure:

(X)ₙ-(A)ₘ-P-(B)ₒ-(Y)ₚ

wherein, P is a PADRE peptide of the invention; X and Y can be the same or different and are antigenic determinants, e.g., carbohydrate epitopes, lipids, proteins, peptides, or combinations thereof; and A and B can be the same or different and are linkers. The letters n through p can be either 1 or 0, with the proviso that n and p cannot both be 0, for antigenic determinants. Each of the components of the compositions of the invention is described in more detail, below.

The present invention is useful for eliciting an immune response to antigenic determinants, typically, a humoral response to a carbohydrate immunogen.

The nomenclature used to describe peptide compounds follows the conventional practice wherein the amino group is presented to the left (the N-terminus) and the carboxyl group to the right (the C-terminus) of each amino acid residue. In the amino acid structure formulae, each residue is generally represented by standard three letter or single letter designations. The L-form of an amino acid residue is represented by a capital single letter or a capital first letter of a three-letter symbol, and the D-form for those amino acids having D-forms is represented by a lower case single letter or a lower case three letter symbol. Glycine has no asymmetric carbon atom and is simply referred to as "Gly" or G.

The nomenclature used to describe carbohydrates includes the following abbreviations: Ara = arabinosyl; Fru = fructosyl; Fuc = fucosyl; Gal = galactosyl; GalNAc = N-acetylgalacto; Glc = glucosyl; GlcNAc = N-acetylgluco; Man = mannosyl; and NeuAc = sialyl (N-acetylneuraminyl).

Carbohydrates are considered to have a reducing end and a non-reducing end, whether or not the saccharide at the reducing end is in fact a reducing sugar. In accordance with accepted nomenclature, carbohydrates are depicted herein with the nonreducing end on the left and the reducing end on the right.

All carbohydrates herein are described with the name or abbreviation for the non-reducing saccharide (*e.g.*, Gal), followed by the configuration of the glycosidic bond (α or β), the ring bond, the ring position of the reducing saccharide involved in the bond, and then the name or abbreviation of the reducing saccharide (e.g., GIcNAc). The linkage between two sugars may be expressed, for example, as 2,3, 2-3, or (2,3). Each saccharide is a pyranose.

### 1. Pan Dr-binding Peptides

The present invention provides methods useful for identification of modifications to a starting peptide which broaden its specificity. For instance, International Application WO 92/02543 describes methods suitable for identifying peptides capable of binding DR molecules. WO 92/02543 describes the use of hemagglutinin from the influenza virus ("HA"), as the source of peptides specifically reacting with HLA-DR. Portions of the protein are screened for reactivity to provide sequences which bind the appropriate DR molecule, such as DR1, DR4w4 or DR4w14.

Once an immunogen or peptide thereof which binds to the selected MHC molecule is identified, a "core binding region" of the antigen or peptide may be determined by synthesizing overlapping peptides, and/or employing N-terminal or C-terminal deletions (truncations) or additions. In the determination of a core binding region and critical contact residues, a series of peptides with single amino acid substitutions may be employed to determine the effect of electrostatic charge, hydrophobicity, etc. on binding.

Within the core region, "critical contact sites," i.e., those residues (or their functional equivalents) which must be present in the peptide so as to retain the ability to bind an MHC molecule and inhibit the presentation to the T cell, may be identified by single amino acid substitutions, deletions, or insertions. In addition, one may also carry out a systematic scan with a specific amino acid (*e.g.*, Ala) to probe the contributions made by the side chains of critical contact residues.

The peptides of the invention are relatively insensitive to single amino acid substitutions with neutral amino acids, except at essential contact sites, and have been found to tolerate multiple substitutions. Particularly preferred multiple substitutions are small, relatively neutral moieties such as Ala, Gly, Pro, or similar residues. The substitutions may be homo-oligomers or hetero-oligomers. The number and types of residues which are substituted or added depend on the spacing necessary between essential contact points and certain functional attributes which are sought (*e.g.*, hydrophobicity versus hydrophilicity). Increased binding affinity for an MHC molecule may also be achieved by such substitutions, compared to the affinity of the parent peptide. In any event, such "spacer" substitutions should employ amino acid residues or other molecular fragments chosen to avoid, for example, steric and charge interference which might disrupt binding.

The effect of single amino acid substitutions may also be probed using D-amino acids. Such substitutions may be made using well known peptide synthesis procedures, as described in e.g., Merrifield, *Science* **232**:341-347 (1986), Barany and Merrifield, The Peptides, Gross and Meienhofer, eds. (N.Y., Academic Press), pp. 1-284 (1979); and Stewart and Young, Solid Phase Peptide Synthesis, (Rockford, Ill., Pierce), 2d Ed. (1984).

The peptides employed in the subject invention need not be identical to peptides disclosed in the Example sections below, so long as the subject compounds are able to bind to the appropriate MHC molecules or provide for humoral or cytotoxic T lymphocytic activity against the target immunogen. Thus, one of skill will recognize that a number of conservative substitutions can be made without substantially affecting the activity of the peptide. Conservative substitutions in which an amino acid residue is replaced with another which is biologically and/or chemically similar, *e.g.*, one hydrophobic residue for another, or one polar residue for another. The substitutions include combinations such as Gly, Ala; Val, Ile, Leu, Met; Asp, Glu; Asn, Gln; Ser, Thr; Lys, Arg; and Phe, Tyr.

In addition, the peptide or oligopeptide sequences can differ from the natural sequence by being modified by terminal-NH₂ acylation, e.g., by alkanoyl (C₁-C₂₀) or thioglycolyl acetylation, terminal-carboxy amidation, e.g., ammonia, methylamine, etc. In some instances these modifications may provide sites for linking to a support or other molecule.

Another approach may be used in which anchor residues that contain side chains critical for the binding to the MHC are inserted into a poly-alanine peptide of 13 residues. This approach has been used by Jardetzky, *et al*., *Nature* **353**:326-329 (1990). They demonstrated that a polyalanine peptide which was modified with a single dominant MHC contact residue (Tyr) endowed the peptide with high affinity binding capacity for DR1. Instead of using tyrosine as the main MHC contact residue, cyclohexylalanine or phenylalanine can also be utilized. These residues are interchangeable with Tyr with respect to a peptide's capacity to bind those DR alleles capable of high affinity binding of the HA peptide, and furthermore also allow binding to MHC molecules that contain a G-V substitution at residue 86 in the DR β chain. This change affects the binding specificity of the B binding pocket in class II MHC such that tyrosine is no longer capable of effective binding, whereas cyclohexylalanine, as well as phenylalanine, can bind.

The biological activity of the peptides identified above may be assayed in a variety of systems. Typically, the ability to inhibit antigen-specific T cell activation is tested. In one exemplary protocol, an excess of peptide is incubated with an antigen-presenting cell of known MHC expression, (e.g., DR1) and a T cell clone of known antigen specificity (e.g., tetanus toxin 830-843) and MHC restriction (again, DR1), and the immunogenic peptide itself (i.e., tetanus toxin 830-843). The assay culture is incubated for a sufficient time for T cell proliferation, such as four days, and proliferation is then measured using standard procedures, such as pulsing with [³H]-thymidine during the last 18 hours of incubation. The percent inhibition, compared to the controls which do not receive peptide, is then calculated.

The capacity of peptides to inhibit antigen presentation in an *in vitro* assay has been correlated to the capacity of the peptide to inhibit an immune response *in vivo. In vivo* activity may be determined in animal models, for example, by administering an immunogen known to be restricted to the particular MHC molecule recognized by the peptide, and the immunomodulatory peptide. T lymphocytes are subsequently removed from the animal and cultured with a dose range of immunogen. Inhibition of stimulation is measured by conventional means, e.g., pulsing with [³H]-thymidine, and comparing to appropriate controls. Certain experimental details will of course be apparent to the skilled artisan. See also, Adorini, *et al., Nature* **334**:623-625 (1988).

A large number of cells with defined MHC molecules, particularly MHC Class II molecules, are known and readily available from, for instance, the American Type Culture Collection ("Catalogue of Cell Lines and Hybridomas," 6th edition (1988)) Rockville, Maryland, U.S.A.

A preferred embodiment of the peptides of the present invention comprises modifications to the N- and C-terminal residues. As will be well understood by the artisan, the N- and C-termini may be modified to alter physical or chemical properties of the peptide, such as, for example, to affect binding, stability, bioavailability, ease of linking, and the like.

Modifications of peptides with various amino acid mimetics or d-amino acids, for instance at the N- or C- termini, are useful for instance, in increasing the stability of the peptide *in vivo*. Such peptides may be synthesized as "inverso" or "retroinverso" forms, that is, by replacing L-amino acids of a sequence with D-amino acids, or by reversing the sequence of the amino acids and replacing the L-amino acids with D-amino acids. As the D-peptides are substantially more resistant to peptidases, and therefore are more stable in serum and tissues compared to their L-peptide counterparts, the stability of D-peptides under physiological conditions may more than compensate for a difference in affinity compared to the corresponding L-peptide. Further, L-amino acid containing peptides with or without substitutions can be capped with a D-amino acid to inhibit exopeptidase destruction of the immunogenic peptide.

Stability can be assayed in a number of ways. For instance, peptidases and various biological media, such as human plasma and serum, have been used to test stability. See, e.g., Verhoef, *et al., Eur. J. Drug Metab. Pharmacokin.* **11**:291-302 (1986); Walter, *et al., Proc. Soc. Exp. Biol. Med*. **148**:98-103 (1975); Witter, *et al., Neuroendocrinology* **30**:377-381 (1980); Verhoef, *et al., J. Endocrinology* **110**:557-562 (1986); Handa, *et al., Eur. J. Pharmacol.* **70**:531-540 (1981); Bizzozero, *et al.*, *Eur. J. Biochem.* **122**:251-258 (1982); Chang, *Eur. J. Biochem.* **151**:217-224 (1985).

Stability may also be increased by introducing D-amino acid residues at the C-and N-termini of the peptide. Previous studies have indicated that the half-life of L-amino acid-containing peptides *in vivo* and *in vitro,* when incubated in serum-containing medium, can be extended considerably by rendering the peptides resistant to exopeptidase activity by introducing d-amino acids at the C- and N-termini.

The peptides or analogs of the invention can be modified by altering the order or composition of certain residues, it being readily appreciated that certain amino acid residues essential for biological activity, *e.g.*, those at critical contact sites, may generally not be altered without an adverse effect on biological activity. The non-critical amino acids need not be limited to those naturally occurring in proteins, such as Lα-amino acids, or their D-isomers, but may include non-protein amino acids as well, such as β-γ-δ-amino acids, as well as many derivatives of L-α-amino acids. As discussed, a peptide of the present invention may generally comprise either L-amino acids or D-amino acids, but not D-amino acids within a core binding region.

The peptides of the invention can be prepared in a wide variety of ways. Because of their relatively short size, the peptides can be synthesized in solution or on a solid support in accordance with conventional techniques. Various automatic synthesizers are commercially available and can be used in accordance with known protocols. See, for example, Stewart and Young, *Solid Phase Peptide Synthesis*, 2d. Ed., Pierce Chemical Co. (1984), *supra*.

Alternatively, recombinant DNA technology may be employed wherein a nucleotide sequence which encodes an immunogenic peptide of interest is inserted into an expression vector, transformed or transfected into an appropriate host cell and cultivated under conditions suitable for expression. These procedures are generally known in the art, as described generally in Sambrook, *et al., Molecular Cloning, A Laboratory Manual*, Cold Spring Harbor Press, Cold Spring Harbor, New York (1989). Thus, fusion proteins which comprise one or more peptide sequences of the invention can be used to present the appropriate T cell epitope.

As the coding sequence for peptides of the length contemplated herein can be synthesized by chemical techniques, for example, the phosphotriester method of Matteucci, *et al., J. Am. Chem. Soc.* **103**:3185 (1981), modification can be made simply by substituting the appropriate base(s) for those encoding the native peptide sequence. Nucleic acid sequences that encode for appropriate linkers can then be added to the peptide coding sequence and ligated into expression vectors commonly available in the art, and the vectors used to transform suitable hosts to produce the desired fusion protein. A number of such vectors and suitable host systems are now available. For expression of the fusion proteins, the coding sequence will be provided with operably linked start and stop codons, promoter and terminator regions and usually a replication system to provide an expression vector for expression in the desired cellular host. For example, promoter sequences compatible with bacterial hosts are provided in plasmids containing convenient restriction sites for insertion of the desired coding sequence. The resulting expression vectors are transformed into suitable bacterial hosts. Of course, yeast or mammalian cell hosts may also be used, employing suitable vectors and control sequences.

### 2. Antigenic Determinants

An antigenic determinant may be administered with the PADRE peptides of the invention, either linked or admixed with the PADRE peptide, to elicit or enhance an immune response. CTL and carbohydrate epitopes from a number of immunogenic biomolecules can be used in the conjugates of the present invention. The particular immunogen used (*e.g.*, polysaccharides, proteins, glycoproteins, lipids, glycolipids, lipopolysaccharides and the like) is not critical to the invention. For a listing of suitable immunogens for use in the present invention, *e.g.,* BioCarb Chemicals Catalogue; and *The Jordan Report: Accelerated Development of Vaccine 1995* NIH, Bethesda, Maryland, 1995).

Examples of suitable immunogens include those derived from bacterial surface polysaccharides which can be used in carbohydrate-based vaccines. Bacteria typically express carbohydrates on their cell surface as part of glycoproteins, glycoplipids, O-specific side chains of lipopolysaccharides, capsular polysaccharides and the like. Exemplary bacterial strains include *Streptococcus pneumonia, Neisseria meningitidis, Haemophilus influenza, Klebsiella spp*., *Pseudomonas spp*., *Salmonella spp*., *Shigella spp*., and Group B *streptococci*.

A number of suitable bacterial carbohydrate epitopes are described in the prior art (*e*.*g*., Sanders, *et al. Pediatr. Res.* **37**:812-819 (1995); Bartoloni, *et al. Vaccine* **13**:463-470 (1995); Pirofski, *et al*., *Infect. Immun.* **63**:2906-2911 (1995) and International Publication No. WO 93/21948) and are provided below.

### 1: CARBOHYDRATES ASSOCIATED WITH HUMAN TUMORS:

### 1.1 The majority of tumors:

| | |
|---|---|
| Gaβ4GlcβCer | Lactosylceramid |

### 1.2 Melanoma:

| | |
|---|---|
| NeuAcα8NeuAcα3Galβ4GlcβCer 9-0-Ac-GD3 | GD3 |
| NeuAcα8NeuAcα3(GalNAcβ4)Galβ4GlcβCer 9-0-Ac-GD2 | GD2 |

Lactonized forms of these

### 1.3 Colon cancer and other types of cancer:

| | |
|---|---|
| GalNAα-Ser(Thr) (glycoprotein) | Tn-antigen |
| NeuAcα6GalNAcα-Ser(Thr) | Sialyl-Tn-antigen |
| Galβ3GlcNAc | Type 1 chain |
| NeuAcα3Galβ3(Fucα4)GlcNAcβ | Sialyl-Lewis a |
| NeuAcα3Galβ3(Fucα4)[NeuAcα6]GlcNAcβ | Disialyl-Lewis a |
| Galβ3(Fucα4)GlcNAcβGalgβ3(Fucα4)GlcNAβ | Dimer Lewis a |
| NeuAcα3Galβ4(Fucα3)GlcNAcβ | Sialyl-Lewis x |
| Galβ4(Fucα3)GlcNAcβ3Galβ4(Fucα3)GlcNAcβ | Dimer Lewis x |
| Galβ4(Fucα3)GlcNAcβ3Galβ4(Fucα3)G1cNAcβ3 | |
| Galβ4(Fucα3)GlcNAcβ | Trimer Lewis x |
| NeuAcα3-Dimer Lewis x | |
| NeuAcα3-Trimer Lewis x | |
| NeuAcα6-Oligomer Lewis x | |

Lactonized forms of these in the case of sialic acid

### 1.4 Lung cancer and other types of cancer:

| | |
|---|---|
| Galβ4GlcNAcβ3Galcβ4GlcNAcβ | i-antigen (rep. lactosamine) |
| Galβ3(Fucα4)GlcNAcβGalβ4(Fucαt3)GIcNAco | Lewis a - Lewis x |
| Fucαt2Galβ3GalNAcβ4(NeuAcα3)Galβ4GlcβCer | Fuc-GM1 |

Lactonized forms of Fuc-GM1

### 1.5 Burkitt's Lymphoma:

| | |
|---|---|
| Galα4Galβ4GlcβCer | Gb3 |

### 1.6 Breast cancer;

| | |
|---|---|
| Fucα2Galβ3GalNAcβ3Galα4Galβ4GlcβCer Tn-antigen Sialyl-Tn-antigen | Fuc-Globopenta |

### 1.7 Teratocarcinoma:

| | |
|---|---|
| NeuAcα3Galβ3GalNAcβ3Galα4Galβ4GlcβCer | Sialyl-Globopenta |

Lactonized form of the above

### 2: CARBOHYDRATES ASSOCIATED WITH EXPERIMENTAL TUMORS:

### 2.1 Melanoma (hamster):

| | |
|---|---|
| NeuAcα3Galβ4GlcβCer GD3 9-0-Ac-GD3 GD2 | GM3 |

Lactonized forms of these

### 2.2 Melanoma mouse

GM3
Lactonized GM3

### 3: CARBOHYDRATES ASSOCIATED WITH INFECTIOUS AGENTS:

Mannan from *Candida albicans*
Polysaccharide isolates from *Mycobacterium bovis* strain BCG
Lipophosphoglycan from *Leishmania major*
Antigenic polysaccharides from *Salmonella typhimurium*

### 4: CARBOHYDRATES ASSOCIATED WITH HIV:

| | |
|---|---|
| Fucα2Galβ4(Fucα3)GlcNAcβ | Lewis y |
| GalNAca3(Fuca2)Galβ3GlcNAcβ | Blood group A, |
| NeuAcα6GalNAcα-Ser(Thr) | Sialyl Tₙ antigen |
| GalNAcα-Ser(Thr) | Tₙ antigen |

An exemplary viral antigen includes those derived from HIV (e.g., gp120). Exemplary fungal antigens include those derived from *Candida albicans, Cryptococcus neoformans, Coccidoides spp*., *Histoplasma spp*., *and Aspergillus spp.* Parasitic antigens include those derived from *Plasmodium spp*., *Trypanosoma spp*., *Schistosoma spp*., *Leishmania spp.* and the like.

Exemplary carbohydrate epitopes include bur are not limited to the following: Galα1,4Galβ- (for bacterial vaccines); GalNAcα- (for cancer vaccines); Manβ1,2(Manβ1,2)ₙ Manβ- (for fungal vaccines useful against, for example, *Candida albicans)*, where n=O→∞; GalNAcβ1,4(NeuAcα2,3)Galβ1,4GlGlcβ-0-ceramide (for cancer vaccines); Galα1,2(Tyvα1,3)Manα1,4Rhaα1,3Galα1,2(Tyα1,3)Manα1,4Rha- and Galα1,2(Abeα1,3)Manα1,4Rhaα1,3Galα1,2(Abeα1,3)Manα1,4Rhaα1,3Galα1,2 (Abeα1,3)Manα1,4Rha- (both of which are useful against, for example, *Salmonella spp.*)

The carbohydrates used in the present invention can be prepared according to standard procedures, known to those of skill in the art. Typically, the oligosaccharides are prepared from suitable monomeric sugars through the formation of glycosidic linkages or isolated from natural sources and modified as appropriate. For example, a β-glycosyl bond can be formed between one sugar bearing a 1-halo substituent and a second, suitably protected sugar having at least one unprotected hydroxyl group. Such transformation are typically carried out in the presence of silver carbonate (Ag₂CO₃) or silver triflate.

Alternatively and preferably, the glycosidic linkages can be formed by enzymatic means, using methods described WO 96/32492. Briefly, glycosyltransferases such as sialyltransferase can be utilized for the construction of specific glycosidic linkages.

A number of sialyltransferases are known to those of skill in the art. This enzyme transfers sialic acid (NeuAc) to a Gal with the formation of an α-linkage between the two saccharides. Bonding (linkage) between the saccharides is between the 2-position of NeuAc and the 3-position of Gal.

An exemplary α(2,3)sialyltransferase (EC 2.4.99.6) often referred to as sialyltransferase, transfers sialic acid to the non-reducing terminal Gal of a Galβ1→3Glc disaccharide or glycoside. See, Van den Eijnden, *et al., J. Biol. Chem.*, **256**:3159 (1981), Weinstein, *et al., J. Biol. Chem.,* **257**:13845 (1982) and Wen, *et al., J. Biol. Chem.,* **267**:21011 (1992). Another exemplary α-2,3-sialyltransferase (EC 2.4.99.4) transfers sialic acid to the non-reducing terminal Gal of the disaccharide or glycoside. See, Rearick, *et al., J. Biol. Chem.*, **254**:4444 (1979) and Gillespie, *et al., J. Biol. Chem.,* **267**:21004 (1992). Further exemplary enzymes include Gal-β-1,4-GlcNAc α-2,6 sialyltransferase (See, Kurosawa, *et al. Eur. J. Biochem.* **219**:375-381 (1994)).

One of skill in the art will understand that other glycosyltransferases can be substituted into similar transferase cycles as have been described in detail for the sialyltransferase. For instance, the glycosyltransferase can also be, for instance, glucosyltransferases, *e*.*g*., Alg8 (Stagljov, *et al*., *Proc. Natl. Acad*. *Sci. USA* **91**:5977 (1994)) or Alg5 (Heesen, *et al. Eur. J. Biochem*. **224**:71 (1994)). Suitable N-acetylgalactosaminyltransferases include α(1,3) N-acetylgalactosaminyltransferase, β(1,4) N-acetylgalactosaminyltransferases (Nagata, *et al. J. Biol. Chem.* **267**:12082-12089 (1992) and Smith, *et al. J. Biol Chem.* **269**:15162 (1994)) and polypeptide N-acetylgalactosaminyltransferase (Homa, *et al. J. Biol Chem.* **268**:12609 (1993)). Suitable N-acetylglucosaminyltransferases include GnTI (2.4.1.101, Hull, *et al., BBRC* **176**:608 (1991)), GnTII, and GnTIII (Ihara, *et al., J. Biolchem.* **113**:692 (1993)), GnTV (Shoreiban, *et al., J. Biol. Chem.* **268**:15381 (1993)), 0-linked N-acetylglucosaminyltransferase (Bierhuizen, *et al., Proc. Natl. Acad. Sci. USA* **89**:9326 (1992)), and hyaluronan synthase. Suitable mannosyltransferases include α(1,2) mannosyltransferase, α(1,3) mannosyltransferase, β(1,4) mannosyltransferase, Dol-P-Man synthase, OChl, and Pmtl.

Other suitable glycosyltransferase cycles are described in Ichikawa, *et al., J. Am. Chem. Soc.* **114**:9283 (1992), Wong, *et al., J. Org. Chem.* **57**:4343 (1992), DeLuca, *et al., J. Am. Chem. Soc.* **117**:5869-5870 (1995), and Ichikawa, *et al.*, in *Carbohydrates and Carbohydrate Polymers,* Yaltami, ed.. (ATL Press, 1993).

For the above glycosyltransferase cycles, the concentrations or amounts of the various reactants used in the processes depend upon numerous factors including reaction conditions such as temperature and pH, and the choice and amount of acceptor saccharides to be glycosylated. Because the glycosylation process permits regeneration of activating nucleotides, activated donor sugars and scavenging of produced pyrophosphate in the presence of catalytic amounts of the enzymes, the process is limited by the concentrations or amounts of the stoichiometric substrates discussed before. The upper limit for the concentrations of reactants that can be used in accordance with the method of the present invention is determined by the solubility of such reactants.

CTL peptides comprising the appropriate epitopes are synthesized and then tested for their ability to bind to MHC Class I molecules in assays using, for example, purified class I molecules and iodinated peptides and/or cells expressing empty class I molecules by, for instance, immunofluorescent staining and flow microfluorimetry, peptide-dependent class I assembly assays, and inhibition of CTL recognition by peptide competition. Those peptides that bind to the class I molecule are further evaluated for their ability to serve as targets for CTLs derived from infected or immunized individuals, as well as for their capacity to induce primary *in vitro* or *in vivo* CTL responses that can give rise to CTL populations capable of reacting with virally infected target cells or tumor cells as potential therapeutic agents.

The one or more CTL and/or antibody-inducing peptides may be administered with one or more pan DR peptides in a mixture which may or may not involve noncovalent associations between the peptides. For instance, one or more of the peptides may be lipidated. Alternatively, the peptides may be covalently linked to form a PADRE-antigenic determinant.

To facilitate the association of the antigenic determinant with the PADRE peptide, additional amino acids can be added to the termini of the peptides. The additional residues can also be used for coupling to a carrier, support or larger peptide, for reasons discussed herein, or for modifying the physical or chemical properties of the peptide or oligopeptide, or the like. Amino acids such as tyrosine, cysteine, lysine, glutamic or aspartic acid, or the like, can be introduced at the C- or N-terminus of the peptide or oligopeptide. In addition, the peptide or oligopeptide sequences can differ from the natural sequence by being modified by terminal-NH₂ acylation, e.g., by alkanoyl (C₁-C₂₀) or thioglycolyl acetylation, terminal-carboxy amidation, e.g., ammonia, methylamine, etc. In some instances these modifications may provide sites for linking to a support or other molecule.

As with the PADRE peptides, it will be understood that the antigenic determinants may be modified to provide other desired attributes, e.g., improved pharmacological characteristics, while increasing or at least retaining substantially all of the biological activity of the unmodified peptide. For instance, the peptides can be modified by extending, decreasing or substituting in the peptides amino acid sequences by the addition or deletion of amino acids on either the amino terminal or carboxy terminal end, or both, of peptides derived from the sequences disclosed herein. Usually, the portion of the sequence which is intended to substantially mimic a CTL or antibody stimulating epitope will not differ by more than about 20% from the sequence of the target antigenic protein, except where additional amino acids may be added at either terminus for the purpose of modifying the physical or chemical properties of the peptide for ease of linking or coupling, and the like. In situations where regions of the peptide sequences are found to be polymorphic among viral subtypes, it may be desirable to vary one or more particular amino acids to more effectively mimic differing phytotoxic T-lymphocyte epitopes of different viral strains or subtypes.

Within the peptide sequence regions identified by the present invention as containing CTL or antibody epitopes, e.g., HBV specific peptides, there are residues (or those which are substantially functionally equivalent) which allow the peptide to retain its biological activity, *i.e.*, the ability to stimulate a class I-restricted cytotoxic T lymphocytic response against virally infected cells or cells which express viral antigens. These residues can be identified by single amino acid substitutions, deletions, or insertions. In addition, the contributions made by the side chains of the residues can be probed via a systematic scan with a specified amino acid (*e.g.*, Ala). Peptides which tolerate multiple substitutions generally incorporate such substitutions as small, relatively neutral molecules, *e.g.*, Ala, Gly, Pro, or similar residues. The number and types of residues which can be substituted, added or subtracted will depend on the spacing necessary between the essential epitopic points and certain conformational and functional attributes which are sought (*e.g.*, hydrophobicity vs. hydrophilicity). If desired, increased binding affinity of peptide analogues to its MHC molecule for presentation to a CTL can also be achieved by such alterations. Generally, any spacer substitutions, additions or deletions between epitopic and/or conformationally important residues should employ amino acids or other moieties chosen to avoid steric and charge interference which might disrupt binding. Peptides which tolerate substitutions while retaining the desired biological activity may also be synthesized as D-amino acid containing peptides, as described above for PADRE peptides.

The peptides of the invention can be combined via linkage to form polymers (multimers), or can be formulated in a composition without linkage, as an admixture. Where a peptide is linked to an identical peptide, thereby forming a homopolymer, a plurality of repeating epitopic units are presented. For example, multiple antigen peptide (MAP) technology can be used to construct polymers containing both CTL and/or antibody peptides and PADRE peptides. When the peptides differ, *e.g.*, a cocktail representing different viral subtypes, different epitopes within a subtype, different HLA restriction specificities, or peptides which contain T helper epitopes, heteropolymers with repeating units are provided. In addition to covalent linkages, noncovalent linkages capable of forming intermolecular and intrastructural bonds are also contemplated.

### B. Preparation of Conjugates

A variety of means of attaching the PADRE peptide to the antigenic determinant are possible. Ionic interactions are possible through the termini or through the ε-amino group of lysine. Hydrogen bonding between the side groups of the residues and the antigenic determinants are also possible. Finally, conformation interactions between the PADRE peptide and the antigenic determinant may give rise to a stable attachment.

As noted above, antigenic determinants may be covalently linked to the PADRE peptides to prepare conjugates of the invention. Particularly preferred antigenic determinant/PADRE peptide conjugates are linked by a spacer molecule or linker. Alternatively, the antigenic determinant may be attached to the PADRE peptide without a linker.

The spacer or linker is typically comprised of neutral molecules, such as, aliphatic carbon chains, amino acids or amino acid mimetics, which are substantially uncharged under physiological conditions and may have linear or branched side chains. A number of compositions and methods for linking various biomolecules are known to those of skill in the art. The particular method by which a PADRE peptide is covalently linked, for instance, to a carbohydrate epitope is not critical to the invention. Methods suitable for linking PADRE peptides to carbohydrate antigens are disclosed for instance in WO 93/21948.

A number of linkers are well known and are either commercially available or are described in the scientific literature. The linking molecules used in the present invention are preferably of sufficient length to permit the two portions of the molecule to interact independently and freely with molecules exposed to them. In the case of carbohydrate epitopes, the linking molecules are typically 1-50 atoms long. Typically, the linking molecules will be aryl acetylene, ethylene glycol oligomers containing 2-14 monomer units, diamines, diacids, amino acids, or combinations thereof. Other suitable linkers include lipid molecules such as ceramide and amino acid residues to which a different carbohydrate moiety is linked through the amino acid side chain.

The particular linking molecule used may be selected based upon its chemical/physical properties. The linking molecule has an appropriate functional group at each end, one group appropriate for attachment to the reactive sites on the carbohydrate portion and the other group appropriate for attachment to the amino acid/peptide portion. For example, groups appropriate for attachment to the carbohydrate portion are carboxylic acid, ester, isocyanate, alkyl halide, acyl halide and isothiocyanate. Similar groups would be useful for attachment to the amino acid portion. Appropriate selection of the functional group will depend on the nature of the reactive portion of the amino acid or peptide.

In one group of embodiments, alkyl or alkylene groups will be useful as linking groups and will have 1 to 20 carbon atoms, with those containing 3 to 6 carbon atoms being particularly preferred. For instance, linkers comprising polyethylene glycol and related structures can be used. The term "polyethylene glycol" is used to refer to those molecules which have repeating units of ethylene glycol, for example, hexaethylene glycol (HO-(CH₂CH₂O)₅-CH₂CH₂OH). When the term "polyethylene glycol" is used to refer to linking groups, it would be understood by one of skill in the art that other polyethers or polyols could be used as well (i.e, polypropylene glycol or mixtures of ethylene and propylene glycols).

In another group of embodiments, the alkyl or alkylene linking groups will be perfluorinated, rendering them less susceptible to biological degradation. *see*, *U.S.* Patent No. 5,055,562. Particularly preferred linking groups will include aminocaproic acid, 4-hydroxy butyric acid, 4-mercapto butyric acid, 3-amino-1-propanol, ethanolamine, perfluoroethanolamine, and perfluorohydroxybutyric acid. In one group of embodiments, the two portions are linked via a polyethylene glycol moiety.

In the case of linkers between PADRE peptides and other peptides (*e.g.*, a PADRE peptide and a CTL inducing peptide), the spacers are typically selected from Ala, Gly, or other neutral spacers of nonpolar amino acids or neutral polar amino acids. In certain preferred embodiments herein the neutral spacer is Ala. It will be understood that the optionally present spacer need not be comprised of the same residues and thus may be a hetero- or homo-oligomer. Preferred exemplary spacers are homo-oligomers of Ala. When present, the spacer will usually be at least one or two residues, more usually three to six residues. In other embodiments the PADRE peptide is conjugated to the CTL or antibody-inducing peptide, preferably with the PADRE peptide positioned at the amino terminus. The peptides may be joined by a neutral linker, such as Ala-Ala-Ala or the like, and preferably further contain a lipid residue such as palmitic acid or the like which is attached to alpha and epsilon amino groups of a Lys residue ((PAM)₂Lys), which is attached to the amino terminus of the peptide conjugate, typically via Ser-Ser linkage or the like.

The CTL or antibody-inducing peptide may be linked to the PADRE peptide either directly or via a spacer either at the amino or carboxy terminus of the CTL peptide. The amino terminus of either the CTL or antibody inducing peptide or the PADRE peptide may be acylated. In addition, the CTL peptide/PADRE conjugate may be linked to certain alkanoyl (C₁-C₂₀) lipids via one or more linking residues such as Gly, Gly-Gly, Ser, Ser-Ser as described below. Other useful lipid moieties include cholesterol, fatty acids, and the like.

In some embodiments it may be desirable to include in the pharmaceutical compositions of the invention at least one component which assists in priming CTL. Lipids have been identified as agents capable of assisting the priming CTL *in vivo* against viral antigens. For example, steroids such as cholesterol, fatty acids such as palmitic acid residues can be attached to the sulfhydryl group of a cysteine residue, the alpha and epsilon amino groups of a Lys residue and then linked, e.g., via one or more linking residues such as Gly, Gly-Gly-, Ser, Ser-Ser, or the like, to an immunogenic peptide, such as a PADRE peptide. Alternatively, in place of fatty acids, long chain alkyl groups can be linked through an ether linkage to the final amino acid (*e.g.*, a cysteine residue).

The lipidated peptide can be injected, either directly in a micellar form, incorporated into a liposome or emulsified in an adjuvant, *e.g.*, incomplete Freund's adjuvant. In a preferred embodiment a particularly effective immunogen comprises palmitic acid attached to alpha and epsilon amino groups of Lys, which is attached via linkage, *e.g.,* Ser-Ser, to the amino terminus of the immunogenic peptide.

As another example of lipid priming of CTL responses, *E. coli* lipoproteins, such as tripalmitoyl-S-glycerylcysteinlyseryl-serine (P₃CSS) can be used to prime virus specific CTL when covalently attached to an appropriate peptide. See, Deres, *et al., Nature* **342**:561-564 (1989). Peptides of the invention can be coupled to P₃CSS, for example, and the lipopeptide administered to an individual to specifically prime a CTL response to the target antigen. Further, as the induction of neutralizing antibodies can also be primed with P₃CSS conjugated to a peptide which displays an appropriate epitope, the two compositions can be combined to more effectively elicit both humoral and cell-mediated responses to infection.

In the case of PADRE peptides conjugated to carbohydrate epitopes, the lipid moieties may be linked to the opposite terminus of the carbohydrate (*e*.*g*., carbohydrate linked to the C-terminus and lipid linked to the N-terminus). Alternatively, both the lipid and the carbohydrate moieties may be linked to the same end of the peptide. For instance, the two moieties may be linked to the same linker on the N-terminus.

### C. Pharmaceutical Compositions

The compounds of the present invention, and pharmaceutical and vaccine compositions thereof, can be administered to mammals, particularly humans, for prophylactic and/or therapeutic purposes. The present invention can be used to elicit and/or enhance immune responses against immunogens. For instance, CTL/PADRE mixtures may be used to treat and/or prevent viral infection and cancer. Alternatively, immunogens which induce antibody responses (*e.g.*, carbohydrates) can be used. Examples of diseases which can be treated using the present invention include various bacterial infections, viral infections, fungal infections, parasitic infections and cancer.

In therapeutic applications, the present invention is administered to an individual already suffering from cancer, or infected with the microorganism of interest. Those in the incubation phase or the acute phase of the disease may be treated with the present invention separately or in conjunction with other treatments, as appropriate.

In therapeutic applications, a composition of the present invention is administered to a patient in an amount sufficient to elicit an effective CTL response or humoral response to the microorganism or tumor antigen and to cure, or at least partially arrest, symptoms and/or complications. An amount adequate to accomplish this is defined as "therapeutically effective dose." Amounts effective for this use will depend in part on the peptide composition, the manner of administration, the stage and severity of the disease being treated, the weight and general state of health of the patient, and the judgment of the prescribing physician.

Therapeutically effective amounts of the compositions of the present invention generally range for the initial immunization that is for therapeutic or prophylactic administration, from about 1.0 µg to about 10,000 µg of peptide for a 70 kg patient, usually from about 100 to about 8000 µg, and preferably between about 200 and about 6000 µg. These doses are followed by boosting dosages of from about 1.0 µg to about 1000 µg of peptide pursuant to a boosting regimen over weeks to months depending upon the patient's response and condition by measuring specific immune responses.

It must be kept in mind that the compositions of the present invention may generally be employed in serious disease states, that is, life-threatening or potentially life threatening situations. In such cases, in view of the minimization of extraneous substances and the relative nontoxic nature of the conjugates, it is possible and may be felt desirable by the treating physician to administer substantial excesses of these compositions.

Further, the present invention can be used prophylactically to prevent and/or ameliorate bacterial infections, viral infections, fungal infections, parasitic infections and cancer. Effective amounts are as described above. Additionally, one of ordinary skill in the vaccine arts would also know how to adjust or modify prophylactic treatments, as appropriate, for example by boosting and adjusting dosages and dosing regimes.

Therapeutic administration may begin at the first sign of disease or the detection or surgical removal of tumors or shortly after diagnosis in the case of acute infection. This is followed by boosting doses until symptoms are substantially abated and for a period thereafter. In chronic infection, initial high doses followed by boosting doses may be required.

Treatment of an infected individual with the compositions of the invention may hasten resolution of the infection in acutely infected individuals. For those individuals susceptible (or predisposed) to developing chronic infection the compositions are particularly useful in methods for preventing the evolution from acute to chronic infection. Where the susceptible individuals are identified prior to or during infection, for instance, as described herein, the composition can be targeted to them, minimizing need for administration to a larger population.

The present invention can also be used for the treatment of chronic infection and to stimulate the immune system to eliminate virus-infected cells in individuals with latent infections. It is important to provide an amount of compositions of the present invention in a formulation and mode of administration sufficient to effectively elicit and/or enhance an immune response. Thus, for treatment of chronic infection, a representative dose is in the range of about 1.0 µg to about 5000 µg, preferably about 5 µg to 1000 µg for a 70 kg patient per dose. Immunizing doses followed by boosting doses at established intervals, *e*.*g*., from one to four weeks, may be required, possibly for a prolonged period of time to effectively immunize an individual. In the case of chronic infection, administration should continue until at least clinical symptoms or laboratory tests indicate that the viral infection has been eliminated or substantially abated and for a period thereafter.

The pharmaceutical compositions for therapeutic or prophylactic treatment are intended for parenteral, topical, oral or local administration. Typically, the pharmaceutical compositions are administered parenterally, *e.g.*, intravenously, subcutaneously, intradermally, or intramuscularly. Because of the ease of administration, the vaccine compositions of the invention are particularly suitable for oral administration. Thus, the invention provides compositions for parenteral administration which comprise a solution of the peptides or conjugates dissolved or suspended in an acceptable carrier, preferably an aqueous carrier. A variety of aqueous carriers may be used, *e.g.*, water, buffered water, 0.9% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as buffering agents, tonicity adjusting agents, wetting agents and the like, for example, sodium acetate, sodium lactate, sodium chloride, potassium chloride, calcium chloride, sorbitan monolaurate, triethanolamine oleate, etc.

The concentration of compositions of the present invention in the pharmaceutical formulations can vary widely, *i.e.*, from less than about 0.1%, usually at or at least about 2% to as much as 20% to 50% or more by weight, and will be selected primarily by fluid volumes, viscosities, etc., in accordance with the particular mode of administration selected.

The present invention may also be administered via liposomes, which serve to target the conjugates to a particular tissue, such as lymphoid tissue, or targeted selectively to infected cells, as well as increase the half-life of the peptide composition. Liposomes include emulsions, foams, micelles, insoluble monolayers, liquid crystals, phospholipid dispersions, lamellar layers and the like. In these preparations the composition to be delivered is incorporated as part of a liposome, alone or in conjunction with a molecule which binds to, for example, a receptor prevalent among lymphoid cells. These molecules would include monoclonal antibodies which bind to the CD45 antigen, or with other therapeutic or immunogenic compositions. Thus, liposomes filled with a desired composition of the present invention can be directed to the site of lymphoid cells, where the liposomes then deliver the selected therapeutic/immunogenic peptide compositions. Liposomes for use in the invention are formed from standard vesicle-forming lipids, which generally include neutral and negatively charged phospholipids and a sterol, such as cholesterol. The selection of lipids is generally guided by consideration of liposome size, acid lability and stability of the liposomes in the blood stream. A variety of methods are available for preparing liposomes, as described in, e.g., Szoka, *et al., Ann. Rev. Biophys. Bioeng.* **9**:467 (1980), U.S. Patent Nos. 4,235,871, 4,501,728, 4,837,028, and 5,019,369.

For targeting to the immune cells, a ligand to be incorporated into the liposome can include antibodies or fragments thereof specific for cell surface determinants of the desired immune system cells. A liposome suspension containing a composition of the present invention may be administered intravenously, locally, topically, etc. in a dose which varies according to, *inter alia*, the manner of administration, the composition being delivered, and the stage of the disease being treated.

Alternatively, DNA or RNA encoding both one or more PADRE peptides and a polypeptide containing one or more CTL epitopes or antibody inducing epitopes may be introduced into patients to obtain an immune response to the polypeptides which the nucleic acid encodes. Wolff, *et. al.*, *Science* **247**: 1465-1468 (1990) describes the expression of polypeptides which nucleic acids encode.

For solid compositions, conventional nontoxic solid carriers may be used. These may include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more conjugates of the invention, and more preferably at a concentration of 25-75%.

For aerosol administration, the compositions of the present invention are preferably supplied in finely divided form along with a surfactant and propellant. Typical percentages of the composition are 0.01-20% by weight, preferably 1-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1-20% by weight of the composition, preferably 0.25-5%. The balance of the composition is ordinarily propellant. A carrier can also be included, if desired, as with lecithin for intranasal delivery.

In another aspect, the present invention is directed to vaccines which contain as an active ingredient an immunogenically effective amount of a composition of the present invention as described herein. The compositions may be introduced into a host, including humans, linked to its own carrier or as a homopolymer or heteropolymer of active peptide units. Such a polymer has the advantage of increased immunological reaction and, where different peptides are used to make up the polymer, the additional ability to induce antibodies and/or CTLs that react with different antigenic determinants. Carriers are well known in the art, and include thyroglobulin, albumins such as bovine serum albumin, tetanus toxoid, polyamino acids such as poly(lysine:glutamic acid), hepatitis B virus core protein, hepatitis B virus recombinant vaccine and the like. The vaccines can also contain a physiologically tolerable (acceptable) diluent such as water, phosphate buffered saline, or saline, and further typically include an adjuvant. Adjuvants such as incomplete Freund's adjuvant, aluminum phosphate, aluminum hydroxide, or alum are materials well known in the art. And, as mentioned above, immune responses can be primed by conjugating compositions of the present invention to lipids, such as P₃CSS. Upon immunization with a composition as described herein, via injection, aerosol, oral, transdermal or other route, the immune system of the host responds by producing an enhanced immune response, humoral and/or cellular.

Vaccine compositions of the invention are administered to a patient susceptible to or otherwise at risk of disease, to elicit and/or enhance an immune response against an antigenic determinant. Such an amount is defined to be an "immunogenically effective dose," either for therapeutic or prophylactic use. In this use, the precise amounts again depend on the patient's state of health and weight, the mode of administration, the nature of the formulation, etc., but generally range from about 1.0 µg to about 5000 µg per 70 kilogram patient, more commonly from about 10 µg to about 500 µg per 70 kg of body weight.

In some instances it may be desirable to combine the compositions of the present invention with vaccines which induce neutralizing antibody responses to infections and cancers of interest.

The compositions of this invention may also be used to make monoclonal antibodies. Such antibodies may be useful as potential diagnostic or therapeutic agents.

The compositions of the present invention may also find use as diagnostic reagents. For example, a composition of the invention may be used to determine the susceptibility of a particular individual to a treatment regimen which employs the antigenic determinants, and thus may be helpful in modifying an existing treatment protocol or in determining a prognosis for an affected individual. In addition, the compositions of the present invention may also be used to predict which individuals will be at substantial risk for developing chronic infection.

The following examples are offered by way of illustration and not by way of limitation:

### D. Examples

### Example 1: Experimental Procedures

### a. Cell Lines and MHC Purification

Various cell lines were used as sources of purified human and mouse class II molecules. The following Epstein-Barr virus (EBV) transformed homozygous cell lines were used as sources of human HLA class II molecules (Valli, *et al*., *J. Clin. Invest.* **91**:616-628, 1993): LG2 [DB1*0101 (DR1)]; 3107 [DRB1*1501 (DR2w2b)]; MAT [DRB1*0301 (DR3) DBR3*0101 (DR52a)]; PREISS [DRB1*0401 (DR4w4)]; BIN40 [DRB1*0404 (DR4w14)]; SWEIG [DRB1*11011 (DR5)]; PITOUT [DRB1*0701 (DR7)]; PF[DQA1*0301/DQB1*0310 (DQ3.1).

In some instances, transfected fibroblasts were used: L416.3 [DAB5*0101 (DR2w2a)]; TR81.19 [DRB3*0101 (DR52a)]; and L257.6 [DRB4*0101 (Drw53)].

For mouse class II molecules, the following cell lines were used: A20 (IA^{d},IE^{d}) (Sette, *et al*., *Science* **258**:1801-1804, (1992)); CH12 (IA^{k}, IE^{k} (Sette, *et al*., 1992); LS102.9 (IA^{β}) (Wall, *et al*., *Int. Imm*. **4**:773-777, (1992)); and DB27.4 (IA^{b}) (Wall, *et al., J. Immuno*. **152**:4526-4536 (1994)).

### b. Purification of MHC molecules

MHC molecules were purified essentially as described (Gorga, *et al., J. Biol. Chem.* **262**:16087-16094 (1987)). Briefly, human class II molecules were purified by affinity chromatography using the LB3.1 (all DR, Valli, *et al., J. Clin. Invest*. **91**:616-628 (1993)) or the IVD 12 (DQ3.1, Sidney, *et al., J. Immunol*. **152**:4516-4525 (1994)) monoclonal antibodies. Mouse class II molecules were purified by the use of the MKD6 (IA^{d}, Sette, *et al*., *Science* **258**:1801-1804 (1992)); 10.3.6 (IA^{k}, Sette, *et al*., *supra);* 14.44 (IE^{d} and IE^{k}, Sette, *et al., supra);* and Y3JP (IA^{s}, Wall, *et al., Int. Immunol*. **4**:773-777 (1992)) monoclonal antibodies.

### c. Peptide Synthesis

Peptides were synthesized by sequential coupling of N-α-Fmoc-protected amino acids on an Applied Biosystems (Foster City, CA) 430A peptide synthesizer using standard Fmoc coupling cycles (software version 1.40). All amino acids, reagents, and resins were obtained from Applied Biosystems or Nova Biochem (San Diego, CA). Solvents were obtained from Burdick & Jackson. Solid-phase synthesis was started from an appropriately substituted Fmoc-amino acid-Wang resin. The loading of the starting resin was 0.5-0.7 mmol/g polystyrene, and 0.1 or 0.25 meq were used in each synthesis. A typical reaction cycle proceeded as follows: The N-terminal Fmoc group was removed with 25% piperidine in dimethylformamide (DMF) for 5 min, followed by another treatment with 25% in DMF for 15 min. The resin was washed 5 times with DMF. A 4 to 10-fold excess of a preformed 1-hydroxybenzotriazole ester of the appropriate Fmoc-amino acid in an N-methylpyrolidone (NMP) solution of was added to the resin, and the mixture was allowed to react for 30-90 min. The resin was washed with DMF in preparation for the next elongation cycle. The fully protected, resin-bound peptide was subjected to a piperidine cycle to remove the terminal Fmoc group. The product was washed with dichloromethane and dried. The resin was then treated with trifluoroacetic acid in the presence of appropriate scavengers (e.g., 5% (v/v) in water) for 60 min at 20°C. After evaporation of excess trifluoroacetic acid, the crude peptide was washed with diethylether, dissolved in water, and lyophilized. The peptides were purified to >95% homogeneity by reverse-phase HPLC using H₂O/CH₃CN gradients containing 0.8% TFA modifier on a Vydac, 300A pore-size, C-18 preparative column. The purity of the synthetic peptides was assayed on an analytical reverse-phase column and their composition ascertained by amino acid analysis and/or sequencing. The cyclohexylalanine used in the synthetic procedures was purchased from Nova Biochem (San Diego, CA). Palmitylated peptides were produced by coupling palmitic acid on the peptides bound to the resin before cleaving the peptide. Coupling was accomplished by a symmetrical anhydride method, i.e., twofold excess of palmitic acid and one-fold of diisopropylcarbodiimide in dichloromethane for 1 hour.

### d. MHC Peptide Binding Assays

Purified mouse or human class II molecules (5 to 500 nM) were incubated with 5 nM ¹²⁵I-peptides for 48 hours in PBS containing 5% DMSO in the presence of a protease inhibitor mixture. Purified peptides were iodinated using the chloramine-T method (Buus, *et al*., *Science* **235**:1353-1358 (1987)). The final concentrations of protease inhibitors were: 1 nM PMSF, 1.3 mM 1.10 phenanthrolone, 73 µM pepstatin A, 8 mM EDTA, 6 mM N-ethylmaleimide, and 200 µM Nα-p-tosyl-L-lysine chloromethyl ketone. Final detergent concentration in the incubation mixture was 2.6% digitonin (IA^{d} and IA^{k}) or 0.05% NP-40 (all other class II molecules). Class II-peptide complexes were separated from free peptide by gel filtration on Sephadex G-50 or TSK2000 columns, and the fraction of peptide bound to MHC class II molecules was calculated as previously described (Sette, *et al.*, *J. Immunol.* **142**:35-40 (1989)). In preliminary experiments, each of the DR preparations was titered in the presence of fixed amounts of iodinated peptides to determine the concentration of class II molecules necessary to bind 10 to 20% of the total radioactivity. All subsequent inhibition and direct binding assays were then performed using this class II concentration. In the inhibition assays, inhibitory peptides were typically tested at concentrations ranging from 120 µg/mL to 1.2 µg/mL. The data were then plotted, and the dose yielding 50% inhibition was measured. Each peptide was tested in two to four completely independent experiments.

As used herein binding at <50 nM constitutes high affinity and binding at 50-500 nM constitutes intermediate affinity binding.

### e. Inhibition of DR Restricted Peptide Presentation

The capacity of peptides to block the antigen presenting function of MHC was assayed by incubating mitomycin C-treated EBV cells of the appropriate DR with inhibitor peptides in RPMI 1640 (Bio Whittaker, Walkersville, MD) in complete medium containing 10% human serum (Gemini Bioproducts, Inc., Calabasas, CA). The inhibitor peptides were routinely titrated over a range of four ten-fold dilutions starting at a concentration of 150 µg/mL in 96-well U-bottom plates (Costar, Cambridge, MA) containing 5 x 10⁴ cells. Along with inhibitor peptide, assay wells also received suboptimal concentrations of the HA 307-319 peptide (DR1, DR4w4, DR5, DR52b) or HA 307-319, Y₃₀₉>F (DR4w14), or Lol P1 171-190 (DR3) (Sidney, *et al., J. Immunol.* **149**:2634-2640 (1992)), which, in the absence of inhibitor peptides, resulted in 30-50% of the maximal proliferative response. This concentration was routinely 50 to 200 µg/mL. After incubating APC with peptides for 2 hr at 37°C in a 5% CO₂ incubator, 2 x 10⁴ T cells were added to each well. The T cell clones used were Cl 1 (DR1 and DR52b (Krieger, *et al., J. Immunol.* **146**:2331-2340 (1991))); Clone 42.19 (DRw14); Clone JK1 (DR5); and line 132-132 (DR3). The proliferation of the T cells was measured three days later. Briefly, 24 hours after T cell addition, [³H]-thymidine (1 µCi/well) (ICN, Irvine, CA) was added to each well for a final 18 hr incubation. Cells were then harvested onto glass fiber filters (LKB Wallac cell harvester 1295-001, LKB, Gaithersburg, MD), and thymidine incorporation (LKB betaplate counter 1205) was measured. The percent inhibition of antigen presentation was calculated for each dose of inhibitor peptide required to inhibit 50% of the proliferative response.

### EXAMPLE 2: DR Binding Specificity of "Universal" Peptide Epitopes

The binding motifs of several murine and human class II MHC alleles have been defined, and motif analysis by sequencing of naturally processed peptides has also recently been described for various class II types (Rudensky, *et al., Nature* **353**:622-627 (1991); Chicz, *et al*.*, Nature* **358**:764-768 (1992); Hunt, *et al., Science* **256**:1817-1820 (1992); and Rudensky, *et al., Nature* **359**:429-431 (1992)).

In the case of DR molecules in particular, it has been shown (Brown, et *al., Nature* **364**:33-39 (1993)) that a large hydrophobic anchor engaging a corresponding hydrophobic pocket of the MHC binding groove is the most crucial determinant of peptide-DR interactions. Several other anchors play definite, albeit less prominent roles and help determine allelic specificity. Recently it has also been emphasized that the peptide backbone of the C-terminal half of the peptide molecule is engaged in direct hydrogen bonding with the walls of the MHC binding groove.

Although the allele-specific polymorphic residues that line the peptide binding pockets of MHC tend to endow each allele with the capacity to bind a unique set of peptides, there are many instances in which a given peptide has been shown to bind to MHC molecules of different specificities. This has been best documented in the case of the human DR isotype, in which it had been previously noted that several DR alleles appeared to recognize similar motifs, and independently, several investigators reported degenerate binding and/or recognition of certain peptide epitopes in the context of multiple DR types, leading to the concept that certain peptides might represent "universal" epitopes (Busch, *et al., Int. Immunol.* **2**:443-451 (1990); Panina-Bordignon, *et al., Eur. J. Immunol.* **19**:2237-2242 (1989); Sinigaglia, *et al., Nature* **336**:778-780 (1988); O'Sullivan, *et al., J. Immunol.* **147**:2663-2669 (1991); Roache, *et al., J. Immunol.* **144**:1849-1856 (1991); and Hill, *et al., J. Immunol.* **147**:189-197(1991)).

The DR binding capacity of previously described peptides capable of binding more than one DR molecule (HA 307-319, Tr 830-843, CS 378-398, MT 17-31, and HBVnc 50-69 was established using the assay described in Example I, paragraph D. The data obtained (Table II, section A) demonstrate that although these peptides were indeed capable of binding several of the DR molecules tested, they failed to bind to others. For example, the HA 307-319 bound with high (<50 nM) or intermediate (50-500 nM) affinity to DR1, DR4w4, DR5, DR7, and DR2w2a, and weakly to DRw53 (2.2 µM); while no binding was detectable for the remaining four DR specificities. HBVnc 50-69 also bound five of the ten DR specificities tested (DR1, DR2w2b, DR4w4, DR5, and DR2w2a) with high or intermediate affinity. Tr 830-843 and CS 378-398 bound with high or intermediate affinity to four out often DR molecules tested (DR1, DR5, DR7, DR2w2a and DR1, DR4w4, DR5, DR7, respectively) and MT 17-31 bound with high or intermediate affinity to three out often of the DR types.

In conclusion, although these previously described "universal" epitopes bound to several DR types, they were not completely cross-reactive in their binding capacity, in that a maximum of 50% of the DR specificities tested bound a given peptide with high to intermediate affinity.

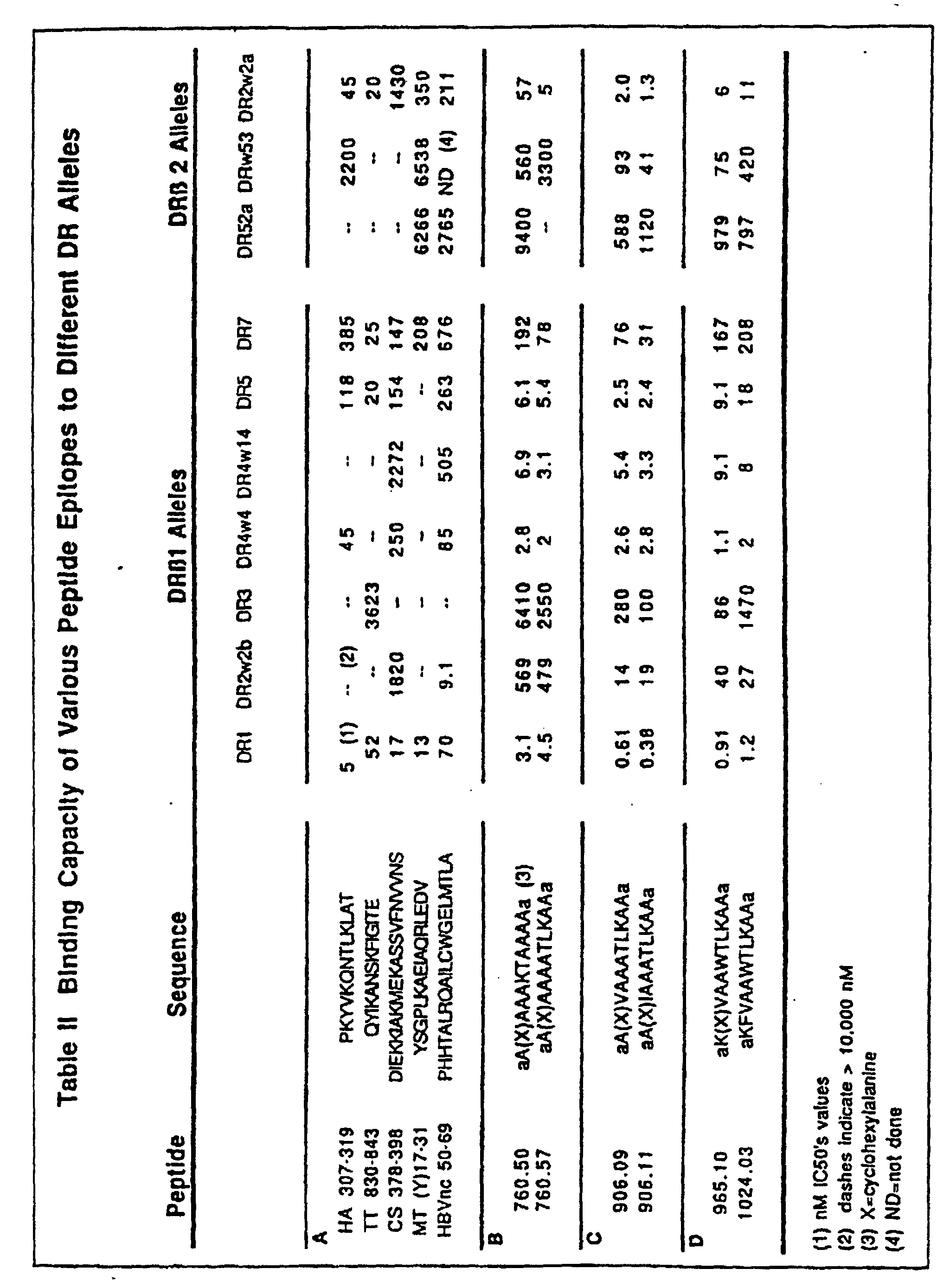

### Example 3: Development of Peptides with High Affinity for Multiple DR Alleles: 760.50 and 760.57

A number of peptides capable of binding with high affinity to the rheumatoid arthritis associated DR alleles DR1, DR4w4, and DR4w14 were generated. To produce these peptides, a strategy was used as initially described by Jardetzky, *et al*., *EMBO J.* **9**:1797-1803 (1990), in which anchor residues that contain side chains critical for the binding to MHC are inserted into a poly-Alanine peptide of 13 residues. Two such peptides, designated 760.50 and 760.57, which are described in copending parent application 08/121,101 were particularly interesting with regard to their broad DR binding specificity. When tested for binding to a panel of 10 purified DR molecules, it was found that, in general, these peptides bound with higher affinity and broader specificity than the natural "universal" epitopes described above (Table II, section B). Neither 760.50 nor 760.57 was completely cross-reactive, since only low affinity binding was detected in 4 of the 10 alleles analyzed (DR2w2b, DR3, DR52a, and DRw53).

### Example 4: Development of Peptides with High Affinity for Multiple DR Alleles: 906-09 and 906.11

To further broaden specificity the 906.09 and 906.11 peptides were synthesized, in which a V or I was introduced at position 4 of the 760.57 peptide. As shown in Table II, section C, both 906.09 and 906.11 peptides retained the good binding affinity for DR1, DR2w2a, DR4, DR5, and DR7 (in the range of 0.3 to 80 nM). Furthermore, the binding capacity (in comparison to 760.50 and 760.57) for molecules DR2w2b, DR3, DR52a, and DRw53 was significantly improved (10- to 25-fold), with IC50 in the range of 20 to 1200 nM. Thus, nine often DR specificities tested bound these peptides with high or intermediate affinity, and one, DR52a, bound weakly.

In conclusion, these data illustrate the development of peptides binding with high affinity to most, if not all, DR alleles. Because of this broad cross-reactivity pattern amongst different DR molecules, we have determined that the 906.09 and 906.11 peptides are PADRE peptides.

### Example 5: Pan DR Binding Peptides Also Bind DQ3.1 and Mouse Class II Molecules

Assays were carried out to determine whether the PADRE peptides were also capable of binding other human class II isotypes or non-human class II molecules. More specifically, the binding capacity of the PADRE peptides to DQ3.1 and several mouse class II specificities was determined, as shown in Table III. For reference purposes, the binding affinities of previously described mouse class II epitopes are also shown in Table III, section A. All of these previously described epitopes bound their relevant restriction elements with high or intermediate affinity, between 20 and 400 nM. It was found that, in general, the 760 series peptides (Table III, section B) bound with intermediate affinity, in the range of 80 to 700 nM, to five of the six alleles tested (IA^{b}, IA^{d}, IE^{d}, IA^{s}, IE^{k}. Interestingly, the 906 series peptides (Table III, section C) bound with significantly higher affinity, in the 10 to 100 nM range in the case of the alleles mentioned above, and 906.11 also bound with intermediate affinity to IA^{k}. With respect to binding to DQ3.1, it was found that 760.50, 760.57, 906.09, and 906.11 all bound with relatively high affinity to purified DQ3.1 molecules (in the 30 to 120 nM range).

As a control, the binding potential of the 760 and 906 peptides to human class I molecules was also examined. No binding was detected, up to 10 µM, to purified HLA-Al, -A2.1, -A3, -A11, and -A24 molecules (data not shown). In conclusion, these data suggest that the 906 series peptides are Pan class II (but not class I) MHC binding, peptides.

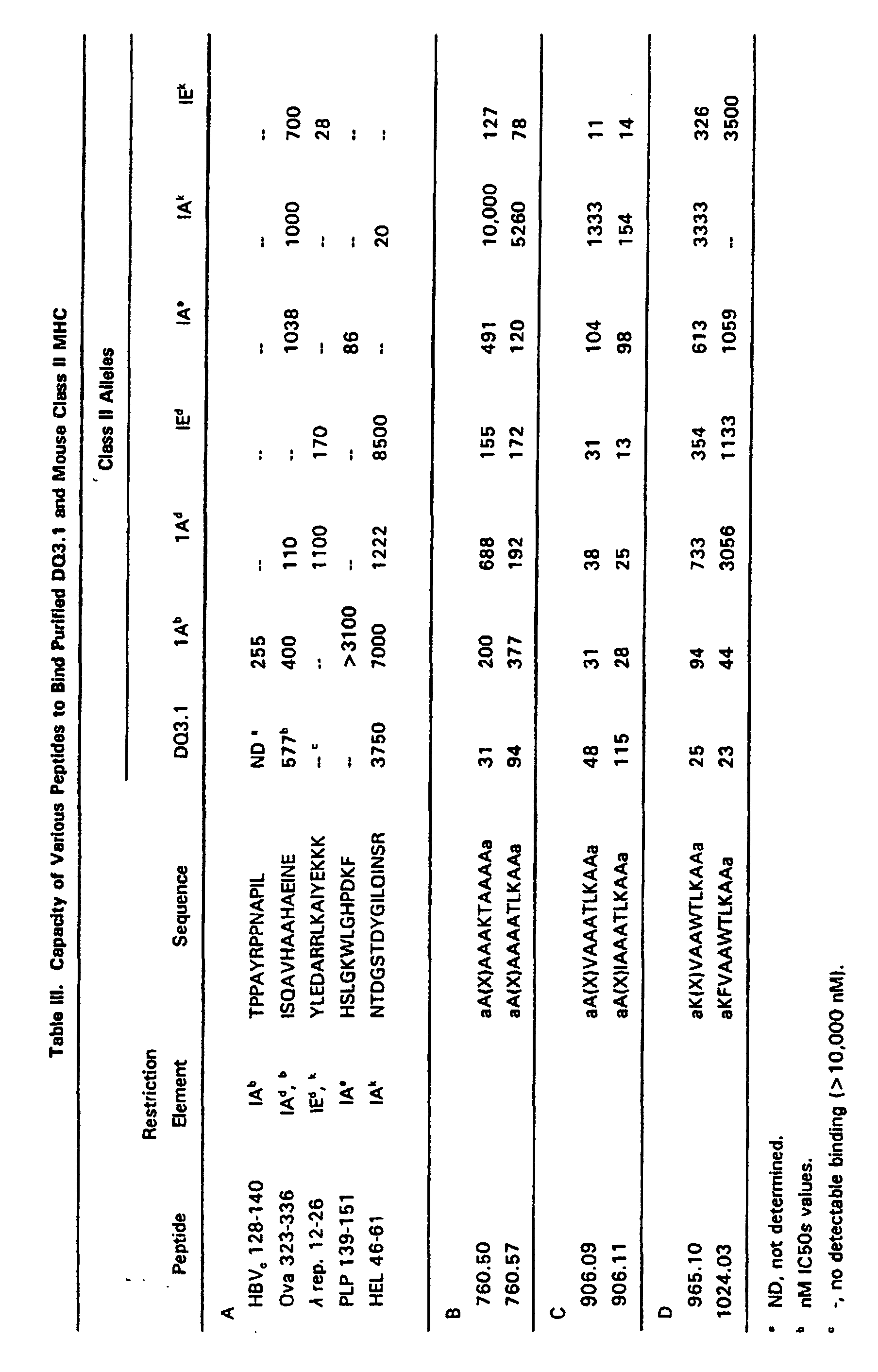

### Example 6: Inhibition of T Cell Proliferation by Pan DR Binders

Because of their degenerate class II binding capacity, the PADRE peptides are candidates as therapeutics in the inhibition of T cell mediated events involved in allograft rejection, allergic responses, or autoimmunity. Accordingly, the capacity of these peptides to block an antigen-specific *in vitro* T cell proliferative response was evaluated. The inhibition of antigen presentation assay described in Example 1, paragraph E was used to make these evaluations.

In keeping with their MHC binding capacity, it was found that these peptides were potent inhibitors of the proliferative responses of human T cells restricted by at least six different DR molecules (Table IV). More specifically, peptides 760.50 and 760.57, which have high binding affinities for DR1, DR4w4, DR4w14, and DR5, inhibited T cell proliferation restricted by those alleles, with IC50 in the 1.0 to 25 µm range. By contrast, these peptides bound DR3 molecules only weakly, in the 2.5 to 6.5 µM range, and accordingly, DR3 restricted T cell activation was inhibited poorly (IC50 of 220 µM for 760.57) or not at all (IC50 of > 250 µM for 760.50).

The 906.09 and 906.11 peptides also inhibited DR1, DR4w4, DR4w14, and DR5 responses quite effectively (IC50 in the 0.5 to 15 µM range). As expected, the 906 analogs, which have intermediate DR3 binding capacity, were also capable of inhibiting DR3 restricted antigen presentation, with IC50 in the 30 to 60 µM range.

In the same set of experiments, we also tested the 760 and 906 peptides for their capacity to inhibit a DR52b restricted response. This experiment was of interest inasmuch as there is not a molecular binding assay to measure peptide binding to DR52b molecules. The data obtained demonstrate that both 906.09 and 906.11 peptides inhibited the presentation of HA 307-319 Clone I in the context of DR52b molecules with good IC50, in the 1 to 2 µM range, thereby extending to an eleventh allele the PADRE capacity of these peptides.

Finally, these peptides failed to inhibit proliferation of the HA-specific, DR-restricted T cell clone in response to the polyclonal mitogen PHA, and also failed to inhibit in the recently described T cell antagonist assay (De Magistris, *et al., Cell* **68**:525-634 (1992)), in which peptides are added subsequent to (not simultaneously with) the antigenic stimulus (data not shown). These findings rule out the possibility that the results described above might have been caused by some non-specific cytotoxicity of the 760 or 906 peptides.

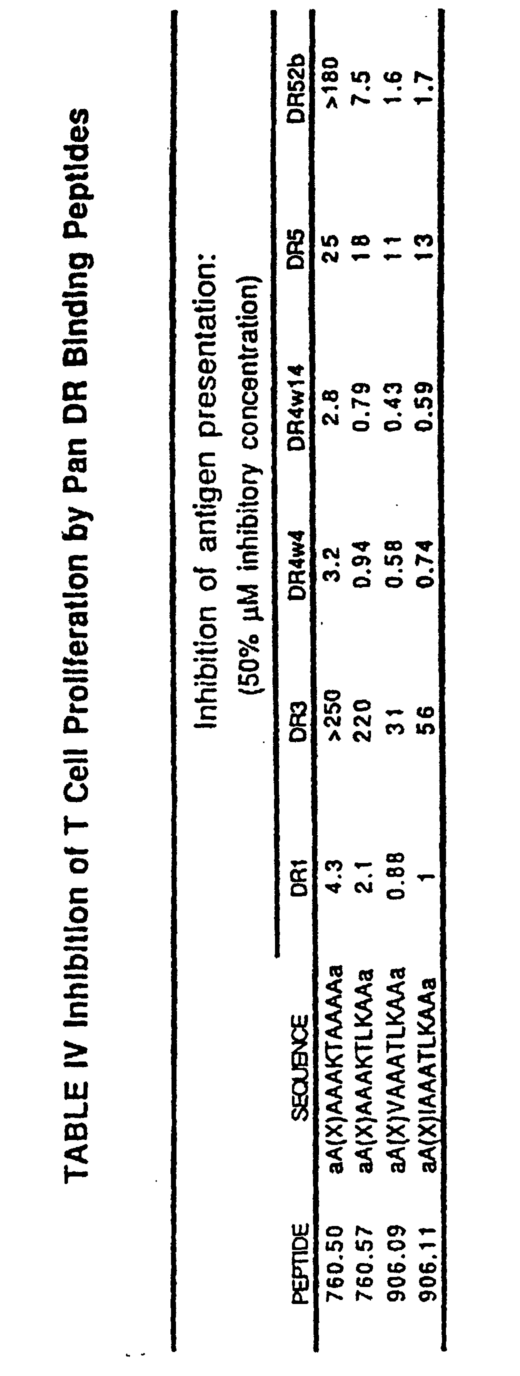

### Example 7: Generation of Pan DR T Cell Epitopes by Modification of Broadly Reactive Class II Binding Peptides

PADRE peptides were used to produce Pan DR restricted T helper epitopes that could provide help for both humoral and cytotoxic responses. Because all of the potential TCR contact residues in the PADRE peptides were alanines, it was hypothesized that due to the limited interactions the methyl side chains could participate in, introduction of more bulky hydrophobic charged residues might improve the likelihood of interactions with T cell receptors and thereby increase their immunogenicity.

Following this line of reasoning, the 906.09 Pan DR peptide was further modified. Several analogs were generated by introducing bulky or charged side groups at positions 2, 5, and 7, which are potential TCR contact residues based on previous analysis of the HA 307-319 peptide. By contrast, those positions known to influence DR binding were left undisturbed (3, 4, 8, 9, and 11). In addition, analogs were also generated that carried the natural amino acid, Phe, instead of cyclohexylalanine at position 3. These peptides were then tested for retention of their capacity to bind multiple DR alleles, and those peptides that had no significant decrease in their DR binding capacity were then tested for their capacity to induce an immune response. The data from two of the best peptides, 965.10 and 1024.03, are discussed below.

When these two peptides were tested for HLA DR and murine Ia binding (Table II, section D and Table III, section D), it was found that, in general, they retained the high binding capacity and broad reactivity associated with the parent peptide, 906.09, for most DR alleles. The exceptions to this were represented by 1024.03 binding only weakly to DR3 (1470 nM) and also binding with intermediate (420 nM) rather than high affinity to DRw53. Also, the 965.10 and 1024.03 peptides showed greatly reduced binding capacities for most of the murine class II molecules tested. Good binding capacity was retained, however, for the 1A^{b} allele, thus allowing H-2^{b} mice to be used to test the *in vivo* immunogenicity of these peptides (see below). Finally, good DQ3. 1 binding capacity of both peptides (in the 25 nM range) was also retained.

### Example 8: In Vitro Immunogenicity of PADRE Peptides

The Pan DR epitope 965.10, along with two of its progenitor peptides, 906.09 and 760.50 and the previously described natural epitope, TT 830-843, were compared for their capacity to stimulate *in vitro* T cell responses in peripheral blood mononuclear cells (PBMC) from normal individuals. The protocol used entailed repeated stimulation of peripheral blood lymphocytes (PBL) with autologous APC and peptide antigens, and was specifically designed to allow the study of primary *in vitro* responses. This protocol is provided below, followed by the results of the assays.

### a. Assay Protocol

PBMC from healthy donors were stimulated *in vitro* using a protocol adapted from Manea, *et al., J. Immunol.* **146**:1964-1971 (1991). PBMC were purified over Ficoll-Paque (Pharmacia LKB, Uppsala, Sweden) and plated in 4 wells of a 24-well tissue culture plate (Costar, Cambridge, MA) at 4 x 10⁶ PBMC/well. The peptides were added at a final concentration of 10 µg/mL. Cultures were then incubated at 37°C, 5% CO₂. On day 4, recombinant IL-2 was added at a final concentration of 10 µg/mL. Cultures were routinely fed every three days thereafter by aspirating off 1 mL of media and replacing it with fresh medium containing IL-2.

T cells (3 x 10⁵/well) were stimulated with peptide (10 µg/mL) using autologous PBMC cells (2 x 10⁶ irradiated (7500 rad)/well) as antigen presenting cells in a total of 3 wells of a 24-well tissue culture plate. Two additional stimulations of the T cells with antigen were performed on approximately days 14 and 28. In addition, on days 14 and 28, T cell proliferative responses were determined as follows: 2 x 10⁴ T cells/well; 1 X 10⁵ irradiated PBMC/well as APC; the peptide concentration was titrated between 0.01-10 µg/mL final concentration in U-bottom 96 well tissue culture plates (Costar, Cambridge, MA). The T cell proliferation assays were harvested on day three as described above.

### b. Results

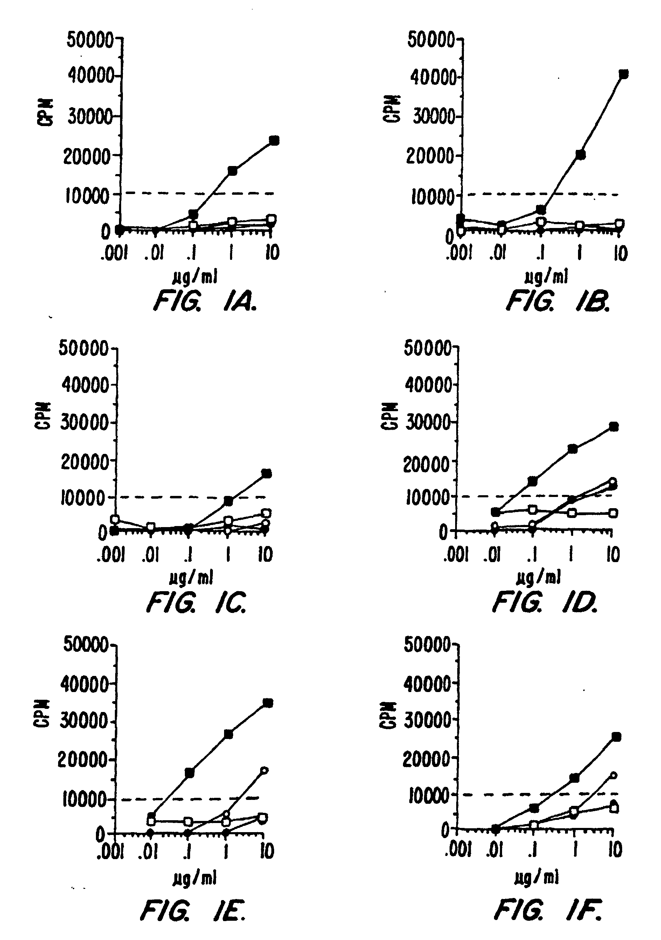

Representative data from three normal donors are shown in Figure 1. The data obtained following two rounds of stimulation are shown in panels A to C, and after a third round of stimulation, in panels D to F. As predicted, the parental peptides 760.50 and 906.09 were poorly immunogenic in these experiments. Neither peptide induced a significant (> 10,000 cpm) response following two rounds of stimulation. After a third round of stimulation, 760.50 induced a response in one donor of the three tested. The natural "universal" epitope TT 830-843 also failed to give a significant response after two rounds of stimulation, and after the third round of stimulation, T-F 830-843 also generated a modest positive response in all three donors. In contrast to these weak responses, all three donors responded briskly after only two rounds of stimulation to the modified Pan DR peptide 965.10.

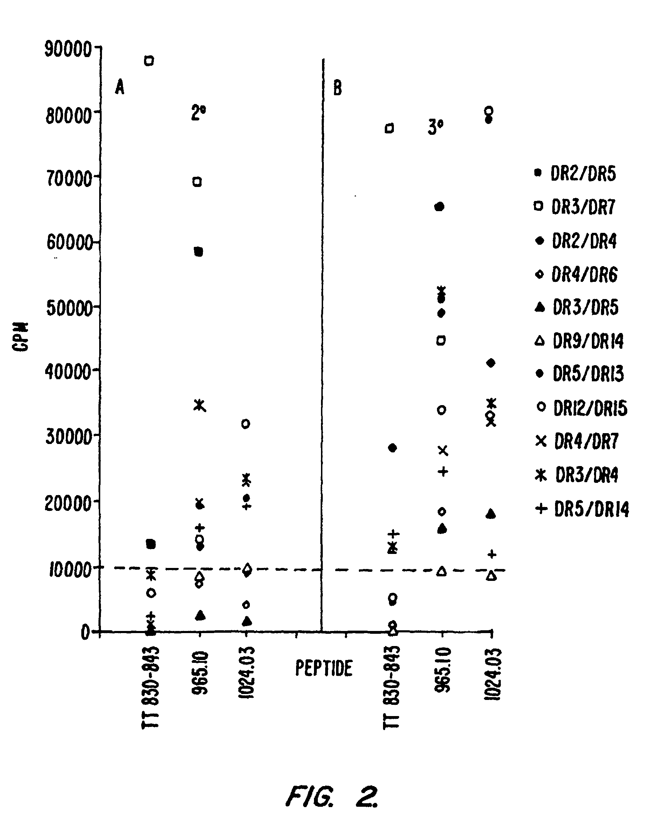

Figures 2A and 2B summarize all the *in vitro* stimulation data that had been obtained in the first series of experiments (second and third stimulations, respectively). After two *in vitro* stimulations (Fig. 2A), peptide 965.10 was the only peptide able to significantly stimulate T cells in the majority of donors (9/12). TT 830-843 was able to generate a response in fewer individuals (3/12), while 760.50 and 906.09 both failed to stimulate any response (0/3). By the third stimulation (Figure 2B), 965.10 generated significant responses in 11 of 12 donors tested, the TT 830-843 was now able to mount a significant response in a majority of the donors (7/12); 760.50 induced a response in 1 of 3 donors, and 906.09 failed to stimulate any of the 3 donors tested.

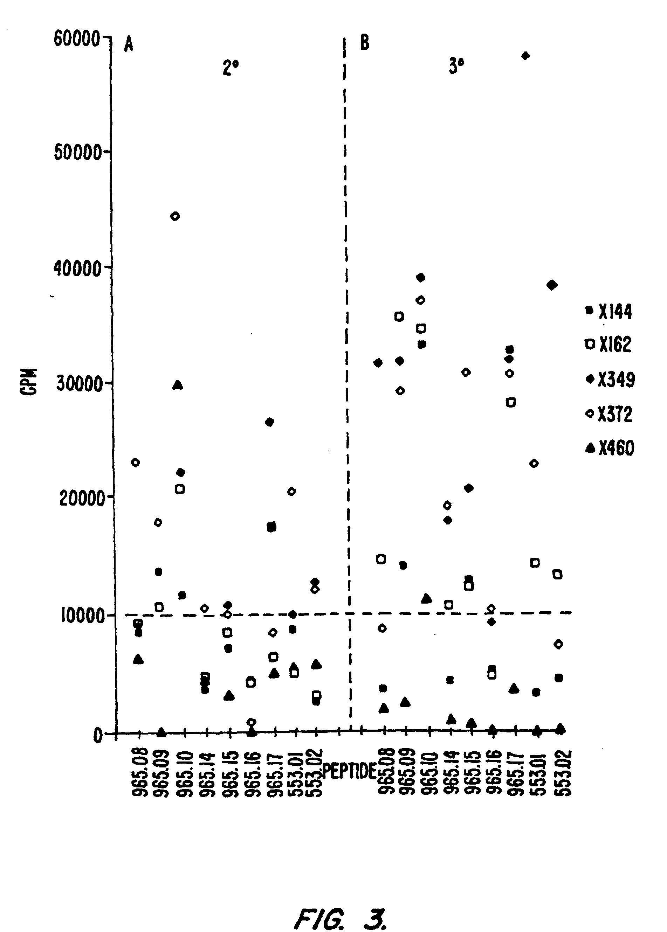

In our next series of experiments, analogs of 965.10 were examined for their ability to induce growth of PBMC. After two *in vitro* stimulations (Fig. 3A), peptide 965.10 was the only peptide able to induce the growth of PBMC cultures above background (10,000 cpm) in all 5 individuals tested. The analogs were, however, successful for a few individuals (965.08 (1/5), 965.09 (4/5), 965.14 (1/5), 965.15 (2/15), 965.16 (0/5), and 965.17 (2/5)). By the third stimulation (Fig. 3B), 965.10 maintained the capacity to induce the growth of PBMC cultures in all 5 individuals. Again the analogs had limited success (965.08 (2/5), 965.09 (4/5), 965.14 (3/5), 965.15 (4/5), 965.16 (1/5), and 965.17 (4/5)). However, two analogs, 965.09 and 965.17, were able to induce the growth of PBMC cultures in 4/5 individuals. It should be noted that as in the previous example, 965.10 was superior to TT 830-843 (553.01) in inducing growth in PBMC cultures.

The ability of 965.10 to induce the growth of specific T cell populations *in vitro* as early as the second stimulation, plus its ability to give a significant T cell response in virtually all of the donors by the third stimulation, demonstrates the superior immunogenic capacity of this peptide relative to peptides TF 830-843, 760.50 or 906.09, 965.08, 965.09, 965.14, 965.15, 965.16, 967.17, or 553.02.

### Example 9: In Vivo Immunogenicity of the Pan DR Epitopes in Mice

The *in vivo* immunogenicity of the 760.50, 965.10, and 1024.03 peptides was tested in C57BL/6J (H-2^{b}+) mice.

To carry out these assays, C57BL/6J mice were injected subcutaneously at the base of the tail with a dose titration of various peptides (0.000125, 0.0025, 0.05, 1, and 20 µg/mouse) in PBS/CFA (Difco, Detroit, MI) in a 100 µl volume. On day 10, inguinal and paraaortic lymph nodes from groups of three mice/peptide dose were collected, pooled, and homogenized into single cell suspensions. Cells were washed two times and subsequently plated (1 x 10⁶ cells/well) in 96-well microtiter tissue culture plates. A log dose peptide titration (0.01 to 100 µg/mL) of the immunizing peptide was added and a standard three day T cell proliferation assay performed as described above.

In these experiments, the activity of the non-natural epitopes to two other previously defined natural IA^{b} restricted epitopes, Ova 323-336 and HBV core 128-140, were compared. These two natural epitopes bound with a somewhat lower (3 to 14 fold) affinity than 965.10 (Table III, section A). The TT 830-843 peptide, which did not bind IA^{b} appreciably (data not shown), was included as a negative control.

As shown in Figure 4, panel A, it was found that, consistent with its inability to bind IA^{b}, TT 830-843 was unable to generate a specific T cell proliferative response. The known IA^{b} restricted helper epitopes Ova 323-336 (Figure 4, panel B) and HBVc 125-140 (Figure 4, panel C) induced responses in the 25,000 to 70,000 cpm range at the two highest peptide doses used for immunization (1 and 20 µg/mouse). The Pan DR epitopes 965.10 (Figure 4, panel D) and 1024.03 (Figure 4, panel E) stimulated the strongest responses, with effective immunizing doses being obtained with as little as 0.05 µg/mouse, with a magnitude in the 100,000 to 150,000 cpm range. In contrast, peptide 760.50 (Figure 4, panel F) was only marginally immunogenic, with a very weak proliferative response being induced, and only at the highest (20 µg/mouse) dose tested. These results indicate that the Pan DR epitopes 965.10 and 1024.03 function as highly effective helper epitopes *in vivo*, as well as *in vitro.* Together with the human immunogenicity data, they also suggest that in addition to high MHC binding capacity, the presence of "immunodominant" amino acid residues at potential TCR contact positions is an important element for the generation of vigorous T cell responses.

### Example 10: Pan DR Peptides Act as Helper Epitopes For In Vivo CTL Induction in Mice

It is generally assumed that the capacity to induce a T cell proliferative response is an indicator of the helper capacity of a peptide epitope. We sought to verify this by measuring the capacity of the 965.10 peptide to deliver help in the generation of a CTL response. The CTL induction experiments were carried out according to the protocol provided below.

### a. CTL induction protocol

Groups of 3-6 C57BL/6J mice were immunized by subcutaneous injection at the base of the tail with a mixture of a lipidated CTL epitope (dipalmitoylated Ova 257-264) and a helper epitope dissolved in phosphate-buffered saline, 5% DMSO. After 11 days, mice were sacrificed and splenocytes (3 x 10⁷/10 mL/T25 flask) were stimulated *in vitro* by the addition of Ova 257-264 (1 µg/mL) and incubated for 6 days. Cytotoxicity was measured using ⁵¹Cr labeled EL4 target cells, which were incubated at 37°C for 1 hour with the CTL epitope peptide. ⁵¹Cr-labeled target cells (10 x 10⁴) were added to varying numbers of effector cells in U-bottomed 96-well plates and ⁵¹Cr release was measured after 6 hours. Data are expressed in lytic units /10⁶ effector cells. One lytic unit is defined as the number of lymphocytes required to achieve 30% lysis of 1 x 10^{4 51}Cr labeled target cells.

### b. Results

The results obtained are shown in Table V. It was found that the Pan DR epitope 965.10 induced a CTL response in a dose-dependent fashion, with an optimum of 307 lytic units observed when 5 nmoles/mouse of 965.10 peptide was co-injected with the lipidated Ova 257-264 CTL epitope. In contrast, the helper activity of the Ova 323-336 and HBVC 128-140 epitopes was much less pronounced, both in terms of the magnitude of the helper effect (four-fold and three-fold increase, respectively) and of the dose required for induction of optimal helper activity (100 nM/mouse).

**Table V.**

| Helper Activity of IA^{b} Restricted Epitopes in CTL Induction | | |
|---|---|---|
| T Helper Peptide | Optimal Dose of Helper Peptide (nmoles/Mouse) | CTL Response (A Lytic Units/106 Cells) |
| - | - | 12+/- 2 |
| Ova 323-336 | 100 | 50 +/- 5 |
| HBV core 128-140 | 100 | 35 +/-lo |
| 965.10 | 5 | 307 +/- 55 |

### Example 11: Induction of Antibodies by PADRE peptides

### a. MATERIALS AND METHODS

### i. Linear constructs

The constructs utilized were mainly based on a dimer of the nine amino acid residue repeat from the repeat region of the recombinant *Plasmodium vivax CS* which contains approximately 60% of the CS protein sequence. This peptide (sequence GDRADGQPAGDRADGQPA, referred to as a B₂ peptide) was shown to be immunogenic in mice of the H-2k and H-2a haplotypes (Nardin, *et al., Eur. J. Immunol* (1988)), but not of the d or b haplotypes.

In addition to the B₂ peptides, we have also used in our experiments two constructs in which the B₂ was covalently linked via its N-terminus to either the PADRE 965.10 peptide (sequence aK(X)VAAWLKAa; x = Cyclohexylamine) or the control IA_{b}-restricted helper epitope OVA 323-336 (sequence ISQAVHAAHAEINIE).

### ii. Polyvalent constructs

It had previously been shown that synthetic vaccines containing sequences which correspond to monovalent immunodominant B cell epitopes, induce only low antibody response and no appreciable protective effect, either by themselves or when coupled to monovalent helper epitopes. Based on these results, recent vaccine designs have focused on the simultaneous incorporation of both multiple B cell epitopes and T helper (Th) epitopes (Tam, *et al., J. Exp. Med.* **171**:229-306 (1990)). The development of the multiple-antigen peptide (MAP) technology (Tam, J.P., *Proc. Natl. Acad. Sci. USA* **85**:5409-5413 (1988)) provides a mean to design such constructs containing both T helper epitopes and multiple B cell sites. Three MAP constructs were used.

### iii. Immunizations

C57B16 (H-2^{b}) mice were injected at the base of the tail with 100 µg of peptide in 100 µl of CFA and bled 4 weeks later. They were subsequently boosted with the same amount of peptide in 100 µl of IFA and bled again 2 weeks later. Antibody titers (primary and secondary response) were measured using an ELISA assay using plates coated with 0.1 µg/well of B₃ peptide (sequence GDRADGQPAGDRADGQPAGDRADGQPA). B10A mice (H-2k) were used as a positive control for anti-B2 peptide response.

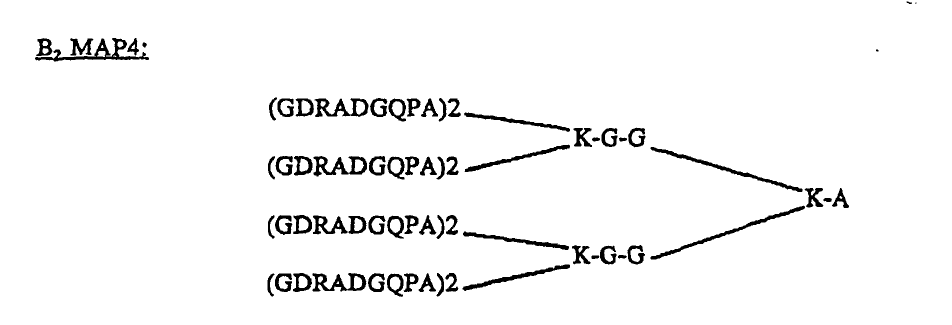

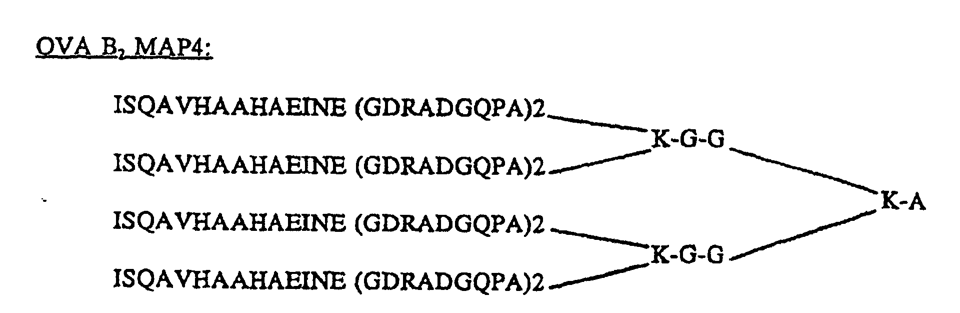

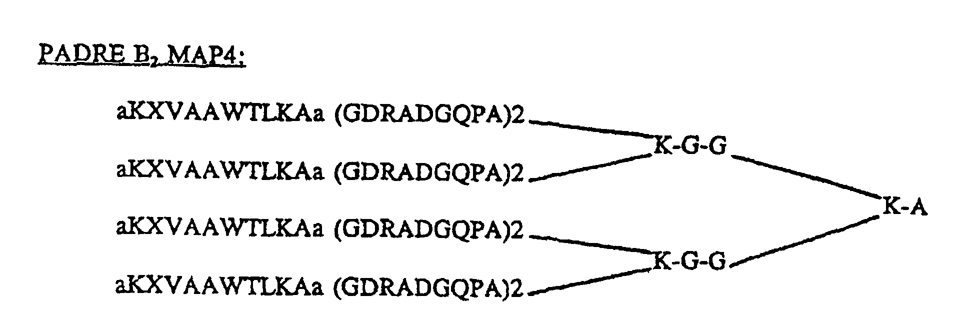

### iv. ELISA

A standard ELISA was performed using (GDRADGQPA)₃ as the coating peptide.

### b. RESULTS

The results obtained (Table VI) show that B10A mice responded very well to the B2 peptide. Similar levels of primary response were observed with either the B₂ or B₂ MAP 4 constructs. Boosting increased the response to the multivalent B₂ MAP 4 about 10 fold, but not to the monovalent B₂ antigen.

In the case of H-2b mice, no response was observed to either B₂ or B₂ MAP 4, either after a single injection or boosting. The MAP constructs which included the OVA or PADRE helper epitopes were remarkably effective, yielding titers in the 1 to 3 x 10⁵ range after a single injection and in the 3 to 8 x 10⁵ range after the boost.

Even the monovalent constructs were somewhat effective if T cell help was provided. The OVA B₂ constructs reached titers in the 3 x 10⁴ range (either after one or two injections). Most interestingly, titers of 6 x 10⁴ and 3.7 x 10⁵ were obtained in even response to the monovalent PADRE B₂ constructs.

**Table VI**

| Mice | | |
|---|---|---|
| Primary Response | | |
| Treatment | B10A(H-2k) | BL6(H-2b) |
| B₂ | 101675*/1.77¹ | 776/1.8 |
| OVA B₂ | ND² | 31963/4.7 |
| PADRE-B₂ | ND | 62764/1.54 |
| B₂ MAP4 | 75755*/1.77 | 90/1.3 |
| OVA-B₂-MAP4 | ND | 257306/3.4 |
| PADRE-B₂-MAP4 | ND | 130113/2.14 |

| Secondary Response | | |
|---|---|---|
| B₂ | 93364*/1.4 | 107/1.34 |
| OVA-B₂ | ND | 37443*/1.8 |
| PADRE-B₂ | ND | 370334/3.25 |
| B₂-MAP4 | 1012119/2.3 | 155/1.17 |
| OVA-B₂-MAP4 | ND | 333850/1.78 |
| PADRE-B₂-MAP4 | ND | 749979/1.79 |

| | | |
|---|---|---|
| Results expressed in geometric mean */Standard deviation of titers from groups of 3 to 6 individual mice. ND indicates Not Done. | | |

### c. CONCLUSION

In conclusion, these experiments demonstrate that PADRE peptides can be utilized to raise powerful antibody responses. Because of their very broad spectrum of reactivity, it is anticipated that use of PADRE peptides should allow for coverage of a very high fraction of the human population, higher than the one covered by either "universal" T cell epitopes. In certain cases, the use of PADRE could also allow the use of simplified constructs, with favorable effects on ease and cost of production and characterization.

### Example 12: PADRE peptides conjugated to Carbohydrates

This example describes the synthesis and use of a PADRE/carbohydrate conjugate to induce an antibody response.

### a. Peptide Synthesis

The peptide aKXVAAWTLKAAaZC (X=L-Cyclohexylalanine, Z=Aminocaproic acid) was prepared according to standard solid phase peptide synthesis procedures, following an Fmoc synthesis strategy.

### b. Carbohydrate Synthesis

Carbohydrate synthesis was carried out as shown in Scheme I.

### i. 1,2,3,6-Tetra-O-benzoyl-α-D-galactopyranoside (1).

A solution of galactose (10 g, 55.5 mmole) in 200 mL of pyridine was cooled to 0°C under argon, benzoyl chloride (26.2 mL, 225 mmole) diluted in 10mL of pyridine was added dropwise over 15 minutes. The mixture was stirred at 0°C for 2 hours. Ice was then added to the solution, and the reaction mixture diluted with CH₂Cl₂ (500 mL) and washed with 20% H₂SO₄ (300 mL), saturated NaHCO₃ (300 mL), water (500 mL) and dried (Na₂SO₄). After filtration and concentration, the crude product was chromatographed (silica, 10% ethyl acetate/toluene) to yield 10.43 g (31%) of a white solid: R_{f} = 0.3 (silica, 10% ethyl acetate/toluene). ¹H-NMR (CDCl₃) δ 8.10 (d, 2 H, aromatic), 8.00 (t, 4 H, aromatic), 7.86 (d, 2 H, aromatic), 7.80-7.36 (m, 9 H, aromatic), 7.27 (t, 3 H, aromatic), 6.82 (d, J = 3.7 Hz, 1 H, H-1), 6.07 (dd, J = 3.7, 10.7 Hz, 1 H, H-2), 5.88 (dd, J = 2.9, 10.7 Hz, 1 H, H-3), 4.78 (dd, J = 6.3, 10.6 Hz, 1 H, H-6), 4.574.46 (m, 3 H).

### ii. 1-Chloro-2,3,4,6-tetra-O-benzyl-α-D-galactopyranoside (2).

Oxalyl chloride (8.8 mL, 100 mmole) was added very slowly to a solution of 2,3,4,6-tetra- O-benzyl-D-galactopyranose (4.5 g, 8.2 mmole) in DMF (80 mL) at -20°C under argon. The mixture was then allowed to warm to 0°C and after 2 hours, 5 mL of additional oxalyl chloride was added. After an additional three hours, the reaction was diluted with CH₂Cl₂ (150 mL) and washed with ice water (200 mL), saturated NaHCO₃ (150 mL), water (150 mL) and dried (Na₂SO₄). Concentration afforded a crude product that was used without purification; R_{f}=0.8 (silica, 10% ethyl acetate/toluene).

### iii. (2,3,4,6-tetra-O-benzyl-α-D-galactopyranosyl)-(1-4)-O-1,2,3,6-tetra-O-benzoyl-α-D galactopyranoside (3).

A suspension of 4 Å molecular sieve (400 mg), compound 1 (2 g, 3.35 mmole), collidine (1 mL, 7.37 mmole), AgOTf (2.58 g, 10 mmole) in 10 mL of toluene was cooled to -20°C under argon. A solution of compound 2 in 10 mL of toluene was then added dropwise and stirred for 1 hour at -20°C. The reaction was allowed to warm to room temperature overnight, the suspension filtered through celite and the filtrate washed with 1N HCl (50 mL), water (50 mL), saturated NaHCO₃ (50 mL), water (50 mL) and dried (Na₂SO₄). Concentration and chromatography (silica, 2% to 10% ethylacetate/toluene) afforded 3.1 g (82%) of a white solid; R_{f}=0.7 (silica, 5% ethyl acetate/toluene). ¹H-NMR (CDCl₃) δ 8.06 (d, 2 H, aromatic), 7.96 (m, 4 H, aromatic), 7.83 (dd, 2 H, aromatic), 7.62-7.11 (m, 32 H, aromatic), 6.84 (d, J = 3.7 Hz, 1 H, H-1), 6.06 (dd, J = 3.7, 11 Hz, 1 H, H-2), 5.77 (dd, J = 2.6, 11 Hz, 1 H, H-3), 4.97 (d, J = 3.4 Hz, 1 H, H-4), 4.89-4.06 (m, 14 H), 3.97 (s, 2 H, benzyl), 3.37 (dd, J = 7.9, 7.9 Hz, 1 H), 2.88 (dd, 1H).

### iv. (α-D-galactopyranosyl)-(1-4)-O-1,2,3,6-tetra-O-benzoyl-α-D-galactopyranoside (4).

Compound **3** (3.08 g, 2.75 mmole) was dissolved in 30 mL of acetic acid and degassed. Palladium on carbon (0.5 g, 10% by weight) was then added and the mixture was placed under a H₂ atmosphere (balloon) for 3 days. The solution was degassed, filtered through celite and concentrated. The residue was chromatographed (silica, 5% CH₃0H/CH₂Cl₂) to afford 1.48g (70%) of a white solid: R_{f}=0.35 (silica, 5% CH₃OH/CH₂Cl₂)-¹H-NMR (CDCl₃) d 8.09 (d 2 H, aromatic), 8.00-7.91 (m, 4 H, aromatic), 7.83 (d, 2 H, aromatic), 7.65-7.36 (m, 10 H, aromatic), 7.27 (d, 1H, aromatic), 7.25 (d, 1 H, aromatic), 6.82 (d, J = 3.7 Hz, I H, H-1), 6.06 (dd, J = 3.7, 10.9 Hz, 1 H, H-2), 5.81 (dd, J = 2.6, 11 Hz, 1 H, H-3), 5.14 (d, J = 2.9 Hz, 1 H, H-4), 4.77-4.61 (m, 3 H), 4.17-3.95 (m, 5 H), 3.38-3.18 (m, 2 H).

### v. (2,3,4,6-Tetra-O-benzoyi-α-D-galactopyranosyl)-(1-4)-O-1,2,3,6-tetra-O-benzoyl-α-D-galactopyranoside (5).

A solution of compound **4** (1.48 g, 1.95 mmole) in 20 mL of pyridine was cooled to 0°C under argon and benzoyl chloride (1.81 mL, 15.6 mmole) was added dropwise and allowed to warm to room temperature overnight. The solution was then diluted with CH₂Cl₂ (100 mL), washed with 2M H₂SO₄ (100 mL), saturated NaHCO₃ (100 mL), water (100 mL) and dried (Na₂SO₄). Concentration and chromatography (silica, 5% ethyl acetate/toluene) afforded 2.03g (88%) of an oil; R_{f}=0.35 (silica, 5% ethyl acetate/toluene); ¹H-NMR (CDCl₃) δ 8.12 (d, 2 H, aromatic), 8.08-7.94 (m, 5 H, aromatic), 7.88-7.79 (m, 7 H, aromatic), 7.63-7.12 (m, 26 H, aromatic), 6.88 (d, J = 3.8 Hz, 1 H, H-1), 6.26-6.16 (m, 3 H, H-2, H- 2', H- 4'), 5.87 (dd, J = 2,5, 11 Hz, 1 H, H-3), 5.85 (dd, J = 3.4, 10.9 Hz, 1 H, H-3), 5.67 (d, J = 3.6 Hz, 1 H, H-1'), 5.03 (dd, J = 7.6 Hz, 1 H), 4.78 (d, J = 2.6 Hz, 1 H, H-4), 4.64-4.55 (m, 2 H), 4.36-4.31 (m, 1 H), 4.04 (dd, J = 8.4, 10.9 Hz, 1 H), 3.94 (dd, J = 5.6, 11 Hz, 1 H).

### vi. Bromo(2,3,4,6-Tetra-O-benzoyl-α-D-galactopyranosyl)-(1-4)-O-2,3,6-tri-O-benzoyl-α-D-galactopyranoside (6).

Hydrogen bromide (33%) in acetic acid (mL) was slowly poured into a solution of compound 5 (2.03 g 1.73 mmole) in acetic acid (mL) at 0°C. The solution was stirred at room temperature for 1 hour and was then diluted with CH₂Cl₂ (200 mL), washed with ice water (200 mL), saturated NaHCO₃ (200 mL) until neutral and dried (Na₂SO₄). Concentration afforded a pale yellow solid (1.84 g, 94%) that was used directly for the next step; R_{f}=0.4 (silica, 5% ethyl acetate/toluene).

### vii. 2-Bromoethyl (2,3,4,6-Tetra-O-benzoyl-α-D-galactopyranosyl)-(14)-O-2,3,6-tri-O-benzoyl-β-D-galactopyranoside (7).

A solution of compound **6** (1.845 g, 1.63 mmole) dissolved in 10 mL of toluene was added dropwise to a mixture of 2-bromo ethanol (0.49 mL, 6.92 mmole), silver triflate (0.53 g, 2.076 mmole), collidine (114 µL 0. 865 mmole) and toluene (15 mL) at 0°C under argon. The suspension was allowed to warm to room temperature overnight and was washed with 2M H₂SO₄ (100 mL), saturated NaHCO₃ until neutral and dried (Na₂SO₄). Concentration and chromatography (silica, 3% ethyl acetate/toluene) afforded 1.38 g (72%) of a white solid; R_{f}=0.3 (silica, 5% ethyl acetate/toluene). ¹H-NMR (CDCl₃) δ 7.98-7.78 (m, 16 H, aromatic), 7.59-7.20 (m, 19 H, aromatic), 6.21 (dd, J = 3.3, 10.8 Hz, 1 H, H-2'), 6.14 (bd, 1 H, H-4'), 5.88 (dd, J = 7.7, 10.5 Hz, 1 H, H-2), 5.79 (dd, J = 3.6, 10.9 Hz, 1 H, H-3'), 5.61 (d, J = 4.4 Hz, 1 H, H-1'), 5.34 (dd, J = 2.8, 10.6 Hz, 1 H, H-3), 4.98 (bt, 1 H), 4.85 (d, J = 7.6 Hz, 1 H, H-1), 4.70 (dd, J = 6.5, 11.2 Hz, 1 H), 4.57 (d, J = 2.6 Hz, 1 H, H-4), 4.36 (dd, J = 7.5, 11.2 Hz, 1 H), 4.18-4.00 (m, 4 H), 3.93-3.05 (m, 1 H), 3.46-3.39 (m, 2 H).

### viii. 2-Bromoethyl(α-D-galactopyranosyl)-(1,4)-O-β-D-galactopyranoside (8).

Sodium methoxide in methanol (25% by weight, 2 mL) was added dropwise to a solution of compound **7** (1.38 g, 1.18 mmole) in 100 mL of methanol. The mixture was stirred under argon for two days, neutralized with AG 50WX8 (H⁺) resin, filtered and concentrated. The residue was chromatographed (C-18 silica, water to 5% CH₃OH/water) to afford 570 mg (100%) of a white solid; R_{f} = 0.1 (silica, CH₂Cl₂/CH₃OH/water (80/20/1)). ¹H-NMR (D₂O) d 4.95 (d, J = 3.8 Hz, 1 H, H-1'), 4.52 (d, J = 7.1 Hz, 1 H, H-1), 4.37 (dd, J = 6.6, 6.6 Hz, 1 H), 4.25-4.17 (m, 1 H), 4.07-4.00 (m, 3 H), 3.93-3.54 (m, 18 H); MS (FAB+) m/z 471 (M⁺ + Na⁺), 473 (M(⁸¹Br⁺ + Na⁺).

### c. Reaction of 2-Bromoethyl (α-D-galactopyranosyl)-(1-4)-O-β-D-galactopyranoside (8) with the polypeptide aKXVAAWTLKAAaZC.

The peptide was conjugated to the 2-Bromoethyl (α-D-galactopyranosyl)-(1-4)-O-β-D-galactopyranoside by reaction of the sulfhydryl group of the cysteine residue with the bromoethyl group of the glycoside. The peptide residues, aminocaproic acid, and cysteine, together with the ethylene moiety of the glycoside, form a spacer between the disaccharide Gal-Gal and the peptide sequence aKXVAAWTLKAAa.

In a typical procedure 1.27 µmoles of peptide, 2.55 µmoles of bromethyl glycoside and 25 µmoles of CS₂CO₃ were added to 1 mL of carefully degassed, dry dimethylformamide and maintained under an argon atmosphere for the duration of the reaction. The mixture was sonicated for 5 minutes and then stirred at room temperature for 2 hours. The dimethylformamide was evaporated *in vacuo*. The residue was dissolved in 2 mL of 20% aqueous acetic acid and purified by preparative high performance liquid chromatography.

### d. Immunizations

C57BL/6J (H-2^{b}) mice, 8 to 16 weeks old, were injected at the base of the tail with 70 µg/mouse of PADRE-sugar in 100 µl of complete Freund's adjuvant (CFA) (Difco Lab, Detroit, MI) and bled 4 weeks later. They were subsequently boosted with the same amount of peptide-sugar compound in 100 µl of incomplete Freund's adjuvant (IFA) and bled again 2 weeks later. Antibody titers (primary and secondary response) were measured using an ELISA assay.

### e. ELISA

100 µL of carboxyethylthioethyl 4-O-α-OGalactopyranosyl-β-O-Galactopyranoside-BSA (Sigma, St. Louis, MO) conjugated in PBS (10 µg/mL) was added to each well of a 96-well flat bottom plate (Immunol II, Dynatech). After blocking with 0.1% BSA, 0.05% Tween 20 in PBS, serial dilutions of the sera from immunized mice were added and the plates incubated 1 hour at 37°C. The plates were washed with PBS + 0.05% Tween-20 then incubated for 2 hours at room temperature with Biotin Conjugated Goat affinity purified antibody to mouse (whole molecule) IgG (Cappel, Durham, NC), and IgM (Caltag Lab, San Francisco, CA). The washed plates were then incubated with a complex Avidin DH and biotinylated horseradish peroxidase H (Vectastain ABC Kit, Vector Lab, Burlingame, CA). The bound anti-sugar antibody was detected with the TMB Peroxidase substrate (Kirkegaard and Perry Laboratories, Gaithersberg, MD). All measurements were performed in duplicate and the antibody titers were defined as the serum dilution yielding 0.3 O.D. units.

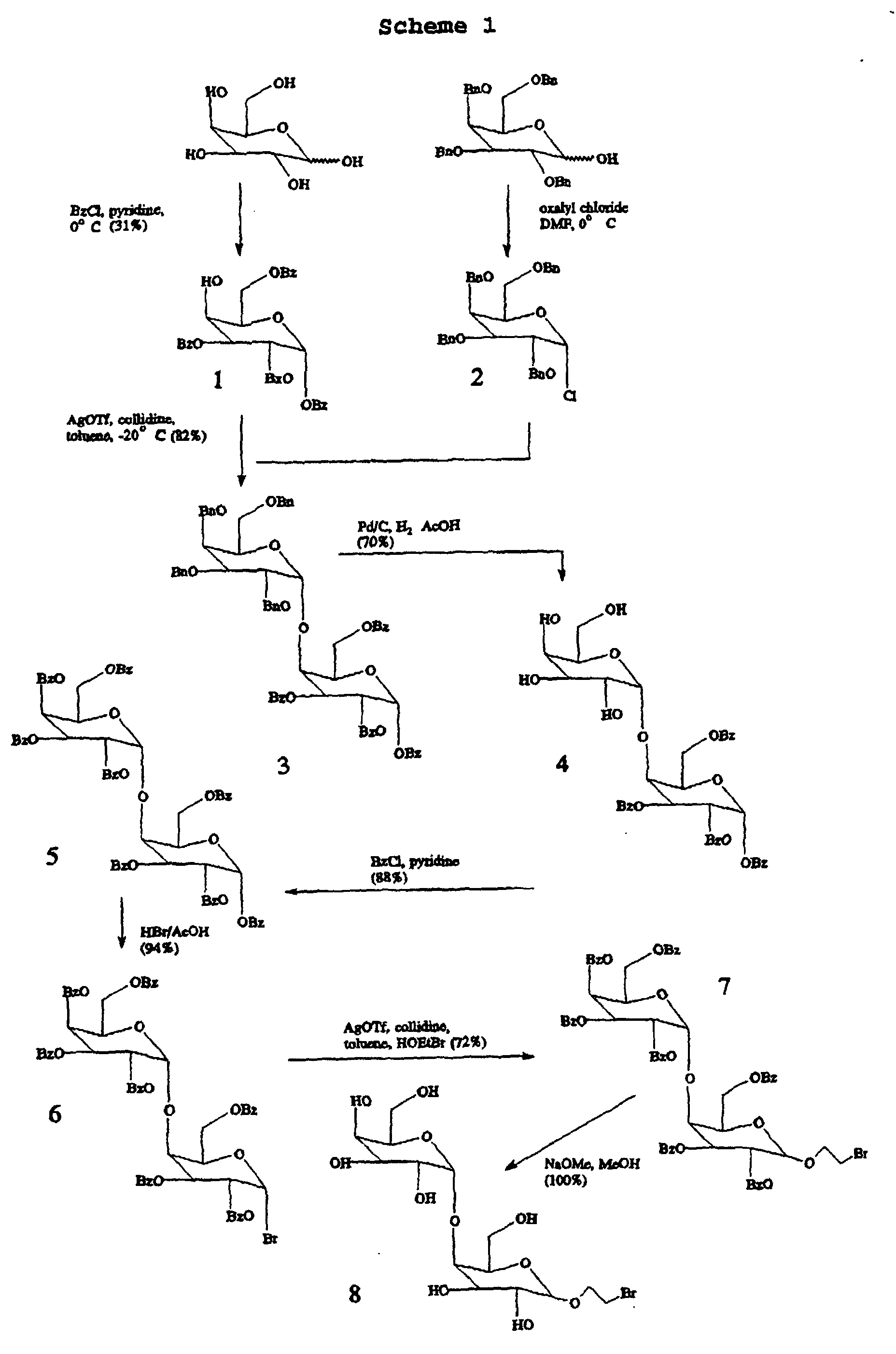

### e. Results

The results obtained (Table VII) show that C57B 1/6J mice responded very well to the disaccharide Gal-Gal. After a single injection, the humoral response was predominately IgG with a mean titer of approximately 4.3 x 10⁴.

**TABLE VII**

| Antibody response to PADRE-sugar 1-10-96 | | |
|---|---|---|
| IMMUNOGEN | ISOTYPES | 1 month after 1st immunization |
| CFA | IgG | 127*/2.24 |
| | IgM | 77*/1.05 |
| | | |
| PADRE-sugar | IgG | 42654*/2.69 |
| | IgM | 100*/11.5 |

### Example 13: Antibody responses to Penta- and Dodecasaccharides

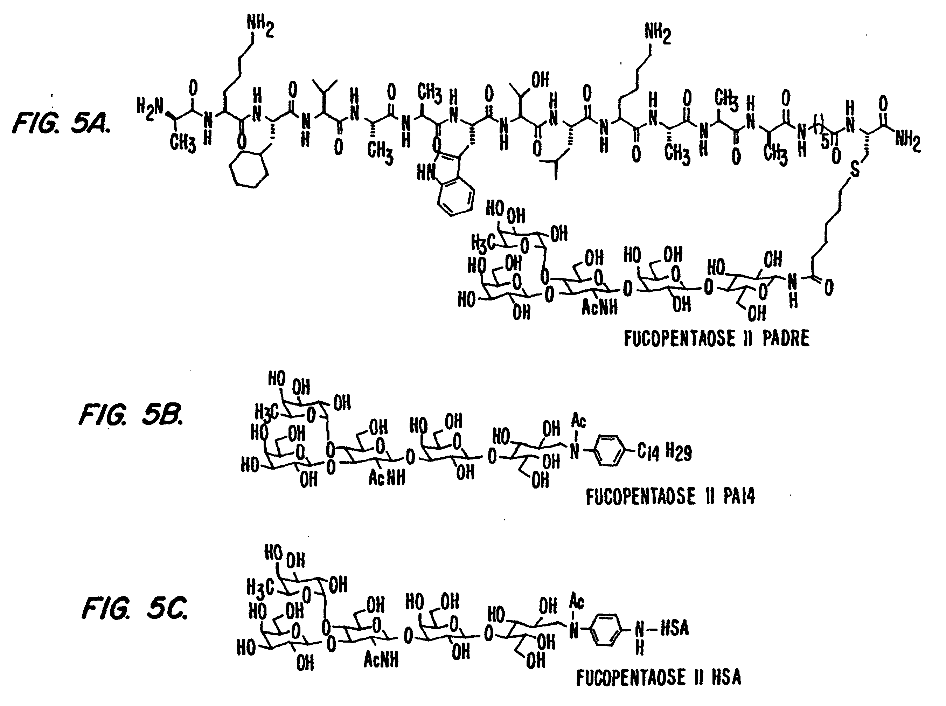

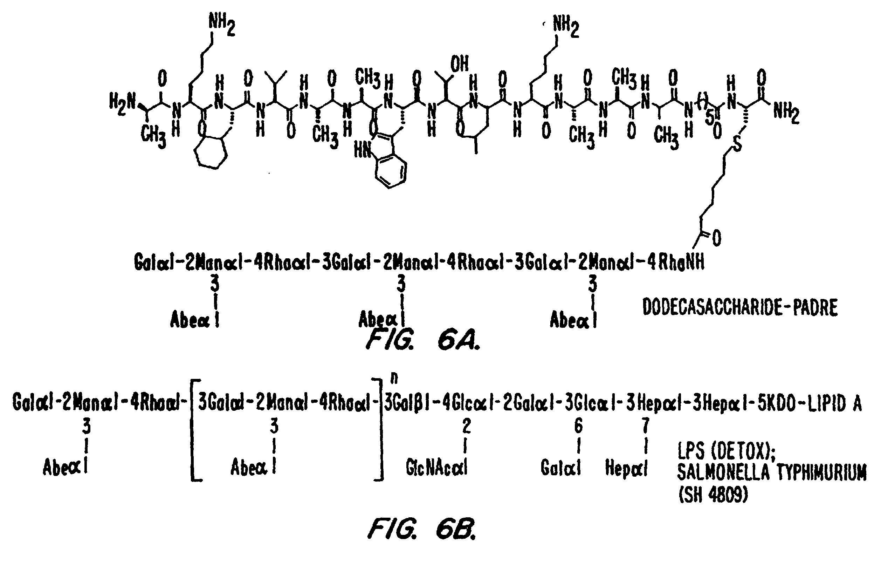

The structures of the saccharides, the saccharide-PADRE conjugates and the immunization controls are given in Figures 5A, B and C; and 6A and B.

### a. CARBOHYDRATE SYNTHESIS

### i. N-6-bromocaproyl β-D-Galactopyranosyl-(1→3)-[α-L-Fucopyrannosyl](1→4)-β-D-2-N-acetimidoglucopyranosyl-(1→3)-β-D-Galactopyranosyl-(1→4)-β-D-glucopyranosylamine (Bromocaproyl Lacto-N-fucopentaose II) (9)

A solution of lacto-N-fucopentaose II (10.0 mg, 11.7 µmol) in saturated NH₄HCO₃ (5 mL) was stirred at room temperature for 3 days with solid NH₄HCO₃ added in fractions during this time to ensure saturation. The mixture was repeatedly lyophilized until a consistent weight was obtained. This yielded a white fluffy solid used directly in the next step without further purification.

The resulting solid was suspended in THF (2.0 mL) and cooled to 4°C. Saturated NaHCO₃ (2.0 mL) was then added to the solution followed by 6-bromocaproyl chloride (20 µL, 131 µmol). After 10 min., an additional portion of 6-bromocaproyl chloride (20 µL, 131 µmol) was added and the stirring was continued at 0°C for 2 hours, and then room temperature for 2 hours. Water (5 mL) was then added to the reaction mixture and the solution was acidified to pH 5 by addition of 1.0 M HCl. After extraction ofthe reaction mixture with diethyl ether (5 mL x 3), the aqueous phase was concentrated to approximately 2 mL and chromatographed (C-18 silica gel, 0% methanol in H₂O to 20% methanol in H₂O). After concentration and lyophilization, a white solid (6.5 mg, 53% yield) was obtained.

¹H NMR (D₂O; 300 MHZ) δ 1.96 (d, 1 H, J = 1.0 Hz), 4.92-4.6 (m), 4.45 (d, 1 H, J = 7.1 Hz), 4.4 (d, J = 7.1 Hz), 4.1 3.35 (m, 30 H), 2.3 (t, 1 H, J = 7.0 Hz). 2.1 (t, 1 H, J = 7.0 Hz), 1.9 (s. 3 H), 1.8 - 1.72 (m, 2 H), 1.60-1.50 (m, 2 H), 1.45-1.32 (m, 2 H), 1.1 (t, J= 7.0 Hz, 3 H).

### ii. N-6-Bromocaproyl α-D-Galactopyranosyl-(1→2)-[α-L-abequosyl](1→3)-α-D-mannopyranosyl-(1→4)-α-D-rhamnosyl-(1→3)-α-D-galactopyranosyl-(1→2)-[α-L-abequosyl](1→3)-α-D-mannopyranosyl-(1→4)-α-D-rhamnosyl-(1→3)-α-D-galactopyranosyl-(1→2)_[α-L-abequosyl](1→3)-α-D-mannopyranosyl-(1→4)-α-D-rhamnosylamine (Bromocaproyl Dodecasaccharide) (10)

A solution of dodecasaccharide (6.0 mg, 3.29 µmol) in saturated NH₄CO₃ (1.5 mL) was stirred at room temperature for 4 days with solid NH₄HCO₃ added in fractions during this time to ensure saturation. Repeated lyophilization of the reaction mixture until a consistent weight afforded a white fluffy solid used directly in the next step without further purification.

The resulting solid was suspended in THF (0.5 mL) and cooled to 0°C. Saturated NaHCO₃ (0.5mL) was then added to the solution followed by 6-bromocaproyl chloride (2 µL, 13.1 µmol). After 10 min., an additional portion of 6-bromocaproyl chloride (8 µL, 542 µmol) was added and the stirring was continued at 0°C. After a further 2 hours, a further portion of the 6-bromocaproyl chloride (8 µL) was added and the reaction mixture was stirred at 0°C for 2 hours, and room temperature for 2 hours. The reaction mixture was then diluted with water (5 mL) and acidified to pH 5 by addition of 1.0 M HCI. This solution was extracted with diethyl ether and the separated aqueous phase was concentrated to about 2 mL and chromatographed (C-18 reverse silica gel, 0% methanol in H₂O to 20% methanol in H₂O). After concentration and lyophilization a white solid was obtained (3.28 mg, 50% yield).

¹H NMR (D₂O), 300 MHZ) δ 5.25 (s), 5.08 (d, 1 H, J = 3.3 Hz), 5.00 (d, 1 H, J = 3.6 Hz), 1.97 (s, 1 ), 4.85 (d, 1 H, J = 2.2 Hz), 4.05-3.30 (m), 2.25 (t, 1 H, J = 7.0 Hz), 210 (t, 1 H, J = 7.3 Hz), 1.90 (br, d, 3 H, J = 8.8 Hz), 1.82-1.74 (m, 2 H), 1.60-1.45 (m, 2 H) 1.4-1.3 (m, 2 H), 1.21 (d, 3 H, J = 6.0 Hz), 1.10-1.02 (m, 6 H); MS (Electrospray) m/z Calcd for C₇₈H₁₃₂ BrNO₅₂: 1995. Found: 1993, 1995.

### b. SACCHARIDE-PADRE CONJUGATION

### i. Lacto-N fucopentaose II-PADRE Conjugate (Pentasaccharide-PADRE)

A mixture of the bromocaproyl lacto-N-fucopentaose (2.3 mg, 2.2 µmol), PADRE peptide (3.1 mg, 2.2 µmol), and cesium carbonate (14 mg, 44 µmol) in anhydrous DMF (1.0 mL, degassed with argon before use) was stirred at room temperature under an argon atmosphere for 24 hours. The reaction mixture was concentrated *in vacuo* and the resulting solid was dissolved in water (0.5 mL). Purification by HPLC (Vydac C-18 column, 25% CH₃CN, 75% H₂O to 35% CH₃CN, 65% H₂O in 55 min, flow rate of 2.0 mL/min) gave the lacto-N-fucopentaose II-PADRE conjugate (2.5 mg, 45% yield).

¹H NMR (D₂O; 300 MHZ) δ 1.96 (d, 1 H, J = 4.0 Hz), 4.92-4.6 (m), 4.45 (d, 1 H, J = 7.1Hz), 4,4 (d, J = 7.1 Hz), 4.1 3.35 (m, 30 H), 2.3 (t, 1 H, J = 7.0 Hz), 2.1 (t, 1 H, J = 7.0 Hz), 1.9 (s, 3 H), 1.8-1.72 (m, 2 H), 1.60-1.50 (m, 2 H), 1.45-1.32 (m, 2 H), 1.1 (t, J = 7.0 Hz, 3 H).

### ii Dodecasaccharide-PADRE Conjugate

A mixture of the bromocaproyldodecasaccharide (1.61 µmol), the PADRE peptide (3.8 mg, 2.46 µmol), and cesium carbonate (10.7 mg, 33 µmol) in anhydrous DMF (1.0 mL, degassed with argon before use) was stirred at room temperature under an argon atmosphere for 14 hours. The reaction mixture was concentrated *in vacuo*. The resulting solid was dissolved in H₂O (1.8 mL), DMSO (0.2 mL) and acetic acid (0.1 mL) and purified by HPLC (Vydac C-18 column, 25% CH₃CN, 75% H₂O to 42% CH₃CN, 58% H₂O in 55 min, flow rate of 2.0 mL/min). After concentration and lyophilization, a white solid (3.1 mg, 54% yield) was obtained.

¹H NMR (DMSO-d6; 500 MHZ) δ 8.52 (d, 1 H, J = 8.07 Hz), 8.18 (d, 1 H, J = 7.67 Hz), 8.10-7.61 (m), 7.75 (d, J = 7.86 Hz), 7.46 (s. 1 H), 7.31 (d, 1 H, J = 8.03 Hz), 7.2-7.5 (m), 7.04 (t, 1 H, J = 7.32 Hz), 6.94 (t, 1 H, J = 7.32 Hz), 5.01-0.80 (m): MS (Electropsray) m/z Calcd for C₁₅₂H₂₅₅N₁₉O₆₉S: 3482 found: 3483 (Mavg), 3505 (M+ Na⁺), 3521 (M+ K⁺).

### c. IMMUNIZATIONS

Three C57BL/6J (H-2^{b}) mice, 8 to 16 weeks old were injected at the base of the tail with 100 µg/mouse of immunogens in 100 µL of CFA and bled 4 weeks later. The mice were subsequently boosted with the same amount of immunogens in 100 µL of IFA and bled again 2 weeks later. The IgG and IgM titers were measured by an ELISA assay.

### d. ELISA

1µg/well of lacto-N-fucopentaose II-HSA (Biocarb Cat. No. 61-03 (1990)) was added to each well of a 96-well flat bottom plate (Immunol II, Dynatech). After blocking with 0.1% BSA, 0.05% Tween 20 in PBS, serial dilutions of the sera from immunized mice were added and the plates incubated 1 hour at 37°C. The plates were washed with PBS + 0.05% Tween- 20 then incubated for 1 hour at room temperature with horse radish peroxidase (HRP)-rat antimouse IgG and an HRP-goat anti-mouse IgM. The bound anti-sugar antibodies was detected with the TMB Peroxidase substrate (Kirkegaard and Perry Laboratories, Gaithersberg, MD). All measurements were performed in duplicate and the antibody titers were defined as the serum dilution yielding 0.3 O.D. units. Antibody responses directed to pentasaccharide immunogens are shown in Table X.

Antibody responses directed to dodecasaccharide immunogens are shown in Table XI. In this ELISA, the wells were coated with 10µg/mL ofLPS. The ELISA reagents used were the same as with the pentasaccharide ELISA. Titers were defined as the serum dilution yielding 0.3 O.D. units.

**Table X:**

| Antibody responses to pentasaccharide (12/17/96) | | | |
|---|---|---|---|
| IMMUNOGENS | Isotypes | Antibody titers 1 month after 1^{st} immunization | Boost |
| CFA | IgG | 0 | 0 |
| | IgM | 38 | 56 |
| | | | |
| Lacto-N- | IgG | 0 | 0 |
| Fucopentaose II- PA14 | IgM | 37 | 50 |
| | | | |
| PADRE-pentaose II | IgG | 3632 | 27968 |
| | IgM | 200 | 198 |

**Table XI:**

| Antibody responses to dodecasaccharide (12/17/96) | | | |
|---|---|---|---|
| IMMUNOGENS | Isotypes | Antibody titers 1 month after 1^{st} immunization | Boost |
| CFA | IgG | 0 | 49 |
| | IgM | 528 | 94 |
| | | | |
| LPS | IgG | 53 | 882 |
| | IgM | 68 | 724 |
| | | | |
| PADRE- | IgG | 7109 | 87435 |
| dodecasaccharide | IgM | 1284 | 698 |

### e. DISCUSSION

The experiments presented herein demonstrate that PADRE peptides can be utilized to raise powerful antibody responses to carbohydrate moieties. When conjugated to pentasaccharides, there is a large increase in the IgG response 1 month after immunization when compared to pentasaccharide alone. And after a boosting injection, the antibody titer shows an even greater increase. Also of importance is the increased IgG response compared to IgM. IgG responses are of a longer term and typically IgG antibodies are of greater affinity.

Similar results are seen with dodecasaccharide PADRE conjugates. There remains a large increase in IgG response when LPS-derived polysaccharide is conjugated to PADRE compared to immunizations with LPS alone. Again, the strength of the IgG response is important in that the response is of a longer term and typically, IgG antibodies are of higher affinity than IgM antibodies.

In conclusion, the experiments presented herein demonstrate that PADRE peptides can be utilized to raise powerful antibody responses to a carbohydrate moiety. Because of their very broad spectrum of reactivity, it is anticipated that use of PADRE peptides should allow for coverage of a very high fraction of the human population, higher than the one covered by either "universal" T cell epitopes, such as TT 830-843 and others (see Alexander, *et al. op. cit*.). The use of PADRE peptides could simplify constructs, with favorable effects on ease and cost of production and characterization. We anticipate that PADRE peptides conjugated to various carbohydrate moieties (sources may include viral, bacterial, fungal tumor, or parasitic) may be developed for diagnostic, prophylactic, or therapeutic disease applications.

### Example 14: Use of Analogs of Known DR Binders to Generate Peptides with Broad Class H Binding Capacity

An alternative approach to generate peptides with broad specificity and high affinity for MHC class II molecules entails using known DR binding peptides and introducing various amino acid changes at positions other than those previously identified as crucial MHC motif residues. Each of these analogs is then tested for class II binding in molecular binding assays as described in the previous examples.

In the set of experiments described herein the peptide 760.57 (aAXAAAATLKAAa) was selected as a starting point for further modification. Peptide 760.57, while not binding appreciably to alleles DR3, DR52a, and DRw53 does however bind well to DR1, 2, 4, 5, and 7. In order not to interfere with the binding capacity to these alleles no substitutions were introduced for the aromatic residue in position 3, for the threonine residue in position 8 and for the alanine residue in position 11. The D-amino acids included at the N- and C-termini to reduce sensitivity to proteolytic degradation (Lamount, *et al.*, *J. Immunol.* **144**: 2493 (1990) were also left unmodified. Four to six amino acid changes were incorporated at other positions (2, 5, 6, 7, 9, 10, and 12) thus generating other 906 series analogs.

Generally, the amino acid substitutions included a positively (K) and negatively (E) charged amino acid, a hydrophilic amino acid (Q), one aliphatic amino acid (V and/or L and/or I), and the aromatic amino acid (F). The binding data obtained when these peptides were tested for binding to a panel of human class II molecules is shown in Table VIII. In general, since the positions crucial for DR1, 2, 4, 5, and 7 binding (positions 3, 8, and 11) had not been modified, good binding capacity was retained. Unless otherwise indicated binding capacity for these alleles was not altered by more than 5 fold. For ease of discussion, in this example, positions are referred to as 1 to 13 from N to C:

### a. Position 2[A]:

The hydrophilic Q (906.03) or the aliphatic V (906.04) substitution induced impressive 30 to 100 fold increases in DR53 reactivity. More modest increases (3-fold) in DR3 reactivity were also induced by the same substitutions. In addition, an approximate 10 fold increase in DQ3.1 binding reactivity was observed for all substitutions tested at this position. Finally, some DR52a reactivity was gained by analogs K (906.01), and Q (906.03).

### b. Position 5[A]:

A modest increase of 2 to 4-fold DR3 binding was accomplished with a V (906.16) or F (906.17). Again impressive (≈ 100-fold) increases were also demonstrated with the same analogs 906.16 and 906.17 for DR53 reactivity. A modest loss (7 fold) of DR1 binding reactivity was observed for a E (906.14) modification. Most modifications at this position resulted (with the exception of F (906.17)) in significant losses of DQ3.1 binding capacity.

### c. Position 6[A]:

A reasonable DR3 reactivity (IC50 = 1500 nM) and some reactivity for DR52a binding were demonstrated with the negatively charged amino acid substitution, E (906.19). An impressive increase in DR53 reactivity (20 fold) was also seen with the same 906.19 analog. However, DR7 reactivity was lost. The K modification (906.18) decreased binding activity of DR1 (8-fold), DR4w14 (10-fold) and DR7 (no detectable binding). The hydrophilic modification Q, (906.20) resulted in loss of DR3 binding and DR7 binding was reduced by 30-fold. Finally, decreases in DQ3.1 binding capacity were also seen with all substitutions tested at this position.

### d. Position 7[A]; 9[L]; and 10[K]:

The only appreciable binding reactivity gained (≈ 10 fold) with substitutions made at these positions was for DQ3.1. The exceptions being 906.29 (K at position -9) and 906.30 (E at position 9) where no reactivity was gained. It is also interesting to note that the analog 906.30 demonstrated a loss of binding reactivities for DR1 (8-fold), DR4w4 (7-fold) DR4w14 (8-fold), DR5 (10-fold), and DR7 (10-fold). Also, it was noted for position 9 that DR3 reactivity was lost for 4 out of 6 substitutions.

### e. Position 12[A]:

Peptide analog 906.50 with an aromatic F substitution increased DR3 and DRw53 reactivities by 8 and 40-fold, respectively. In addition, DR52a binding reactivity was gained with the F substitution.

Second generation (906.51, 906.52, 906.53, 906.55, and 906.56) analogs were also synthesized using combinations of substitutions that in the set of experiments described above, increased DR3 and/or DR52a reactivities (906.04 (V) at position 2; 906.09 (V) at position 4; 906.16 (V) or 906.17 (F) at position 5; and 906.50 (F) at position 12). In general, these second generation analogs also demonstrated good reactivity for most of the DR alleles tested (with the exception of the 906.55 analog binding to DR52a).

In the next series, 965.01 to 965.19, analogs of 906.09 were generated by introducing bulky or charged amino acids at non-critical MHC contact residues. In general, high- or intermediate- capacity binding was maintained (Table IX) for DR1, 2w2β2, 4w4, 4w14, 5, 7 and DRw53. Binding capacity varied for DR3.

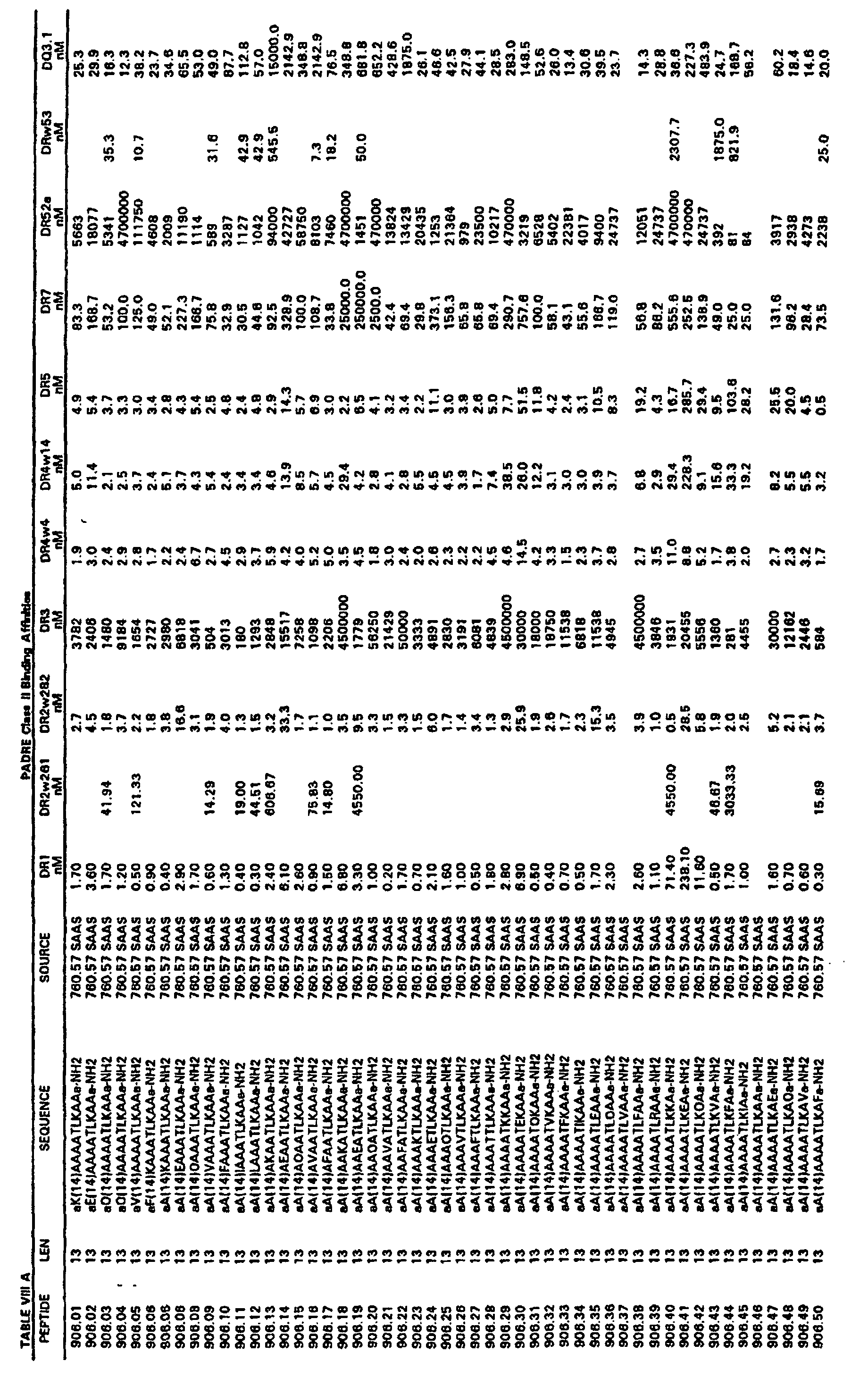

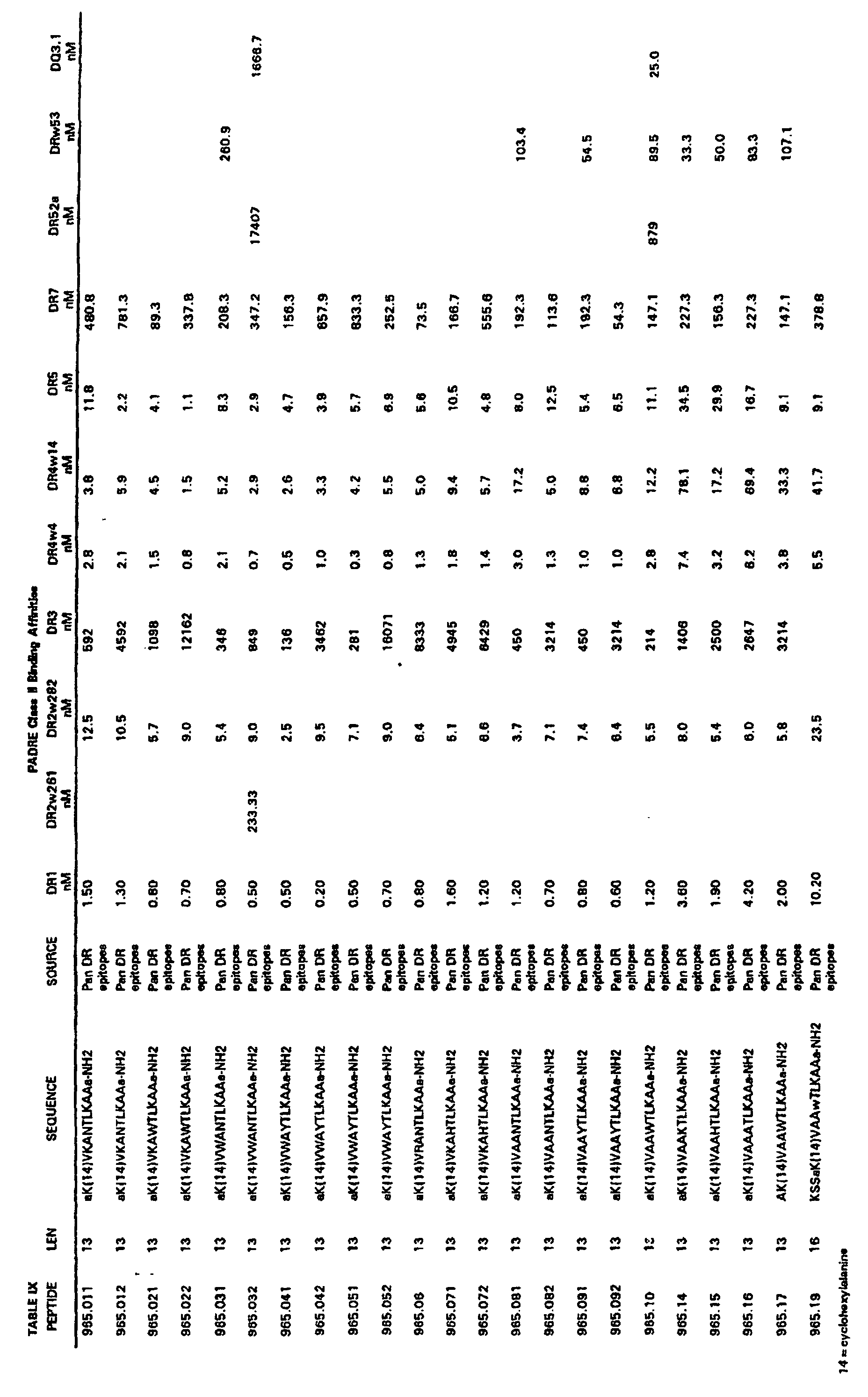

### f. Conclusion

Peptide analogs with broader specificity and higher affinity than known DR binding peptides may be generated by introducing various substitutions at non-critical motif residues, followed by combining these favorable substitutions into next generation peptide analogs.

The above examples are provided to illustrate the invention but not to limit its scope.

## Claims

1. A composition for eliciting an immune response to a non-proteinaceous antigenic determinant, the composition comprising a PADRE oligopeptide of less than about 50 residues and the antigenic determinant.

2. The composition of claim 1, wherein the oligopeptide and the antigenic determinant are attached to each other.

3. The composition of claim 2, wherein the oligopeptide and the antigenic determinant are covalently linked.

4. The composition of claim 3, wherein the antigenic determinant is covalently linked to the PADRE peptide through a linker.

5. The composition of claim 4, wherein the linker comprises a cysteine residue.

6. The composition of claim 4, wherein the linker consists of an aminocaproic acid residue and a cysteine residue.

7. The composition of claim 1, wherein the antigenic determinant is linked to the C-terminus of the oligopeptide.

8. A composition of claim 1, wherein the antigenic determinant comprises a carbohydrate epitope.

9. The composition of claim 8, wherein the carbohydrate epitope is derived from a bacterium.

10. The composition of claim 8, wherein the carbohydrate epitope is derived from a virus.

11. The composition of claim 8, wherein the carbohydrate epitope is derived from a cancer cell.

12. The composition of claim 8, wherein the carbohydrate epitope is derived from a fungus.

13. The composition of claim 8, wherein the carbohydrate epitope is derived from a parasite.

14. The composition of claim 1, wherein the PADRE peptide is further covalently linked to a lipid moiety.

15. The composition of claim 14, wherein the lipid moiety comprises palmitic acid.

16. The composition of claim 15, wherein the lipid moiety is PAM₂K, wherein K is a lysine residue and PAM is a palmitic acid residue.

17. The composition of claim 14, wherein the lipid moiety is linked to the N-terminus of the PADRE peptide.

18. The composition of claim 1, wherein the PADRE peptide has the formula R₁-R₂-R₃-R₄-R₅, proceeding from the N-terminus to the C-terminus, wherein:
R₁ consists of at least 2 residues;
R₂ is selected from the group consisting of a cyclohexylalanine residue, a tyrosine residue, a phenylalanine residue and conservative substitutions therefor;
R₃ is 3 to 5 residues;
R₄ is selected from the group consisting of threonine-leucine-lysine, lysine-threonine, and tryptophan-threonine-leucine-lysine, and conservative substitutions therefor; and
R₅ consists of at least 2 residues.

19. The composition of claim 18, wherein each amino acid of R₃ is independently selected from the group consisting of alanine, isoleucine, serine and valine.

20. The composition of claim 18, wherein each amino acid of R₅ is independently selected from the group consisting of alanine, serine and valine.

21. The composition of claim 18, wherein:
R₁ is D-alanine followed by an L-Alanine or L-Lysine;
R₂ is clyclohexylalanine or phenylalanine;
each residue of R₃ is selected from the group consisting of L-Alanine, isoleucine, and valine; and
R₅ is 2 or 4 L-Alanines followed by D-alanine.

22. The composition of claim 18, wherein:
R₁ is L-Alanine followed by an L-Alanine or L-Lysine;
R₂ is clyclohexylalanine or phenylalanine;
each residue of R₃ is selected from the group consisting of L-Alanine, isoleucine, and valine; and
R₅ is 2 or 4 L-Alanines followed by L-Alanine.

23. The composition of claim 18, wherein the PADRE peptide is selected from the group consisting of aAXAAAKTAAAAa, aAXAAAATLKAAa, aAXVAAATLKAAa, aAXVAAATLKAAa, aAXIAAATLKAAa, aKXVAAWTLKAAa, and aKFVAAWTLKAAa wherein a is d-alanine, A is L-Alanine, X is cyclohexylalanine, K is lysine, T is threonine, L is leucine, V is valine, I is isoleucine, W is tryptophan, and F is phenylalanine.

24. The composition of claim 23, wherein the PADRE peptide is aKXVAAWTLKAAa.

25. The composition of claim 18, wherein the PADRE peptide is selected from the group consisting of AAXAAAKTAAAAA, AAXAAAATLKAAA, AAXVAAATLKAAA, AAXVAAATLKAAA, AAXIAAATLKAAA, AKXVAAWTLKAAA, and AKFVAAWTLKAAA wherein A is L-Alanine, X is cyclohexylalanine, K is lysine, T is threonine, L is leucine, V is valine, I is isoleucine, W is tryptophan, and F is phenylalanine.

26. The composition of claim 18, wherein the PADRE peptide is selected from the group consisting of AAXAAAKTAAAAa, AAXAAAATLKAAa, AAXVAAATLKAAa, AAXVAAATLKAAa, AAXIAAATLKAAa, AKXVAAWTLKAAa, and AKFVAAWTLKAAa wherein a is D-alanine, A is L-Alanine, X is cyclohexylalanine, K is lysine, T is threonine, L is leucine, V is valine, I is isoleucine, W is tryptophan, and F is phenylalanine.

27. The composition of claim 18, wherein the PADRE peptide is selected from the group consisting of aAXAAAKTAAAAA, aAXAAAATLKAAA, aAXVAAATLKAAA, aAXVAAATLKAAA, aAXIAAATLKAAA, aKXVAAWTLKAAA, and aKFVAAWTLKAAA wherein a is D-alanine, A is L-Alanine, X is cyclohexylalanine, K is lysine, T is threonine, L is leucine, V is valine, I is isoleucine, W is tryptophan, and F is phenylaline.

28. A pharmaceutical composition comprising a pharmaceutically acceptable carrier, a non-proteinaceous antigenic determinant and a PADRE oligopeptide of less than 50 residues.

29. The composition of claim 28, further comprising an adjuvant.

30. The composition of claim 29, wherein the adjuvant is Freund's complete adjuvant or Freund's incomplete adjuvant.

31. The composition of claim 28 for use in a method of inducing a humoral immune response comprising introducing into a mammal the composition.

32. The composition of claim 31, wherein the introduction is parenteral.

33. The composition of claim 31, wherein the immune response is prophylactic.

34. The composition of claim 31, wherein the immune response is therapeutic.

35. The composition of claim 31, wherein the immune response comprises an IgG response directed against the antigenic determinant of claim 1.

36. The composition of claim 1, wherein the composition consists of a PADRE oligonucleotide of less than about 50 residues and the antigenic determinant.

## Patentansprüche

1. Zusammensetzung zur Auslösung einer Immunreaktion auf eine nicht proteinische antigene Determinante, **dadurch gekennzeichnet, dass** die Zusammensetzung ein PADRE-Oligopeptid mit weniger als etwa 50 Resten und die antigene Determinante umfasst.

2. Zusammensetzung nach Anspruch 1, **dadurch gekennzeichnet, dass** das Oligopeptid und die antigene Determinante aneinander angelagert sind.

3. Zusammensetzung nach Anspruch 2, **dadurch gekennzeichnet, dass** das Oligopeptid und die antigene Determinante kovalent verbunden sind.

4. Zusammensetzung nach Anspruch 3, **dadurch gekennzeichnet, dass** die antigene Determinante über einen Linker kovalent mit dem PADRE-Peptid verbunden ist.

5. Zusammensetzung nach Anspruch 4, **dadurch gekennzeichnet, dass** der Linker einen Cysteinrest umfasst.

6. Zusammensetzung nach Anspruch 4, **dadurch gekennzeichnet, dass** der Linker aus einem Aminocapronsäurerest und einem Cysteinrest besteht.

7. Zusammensetzung nach Anspruch 1, **dadurch gekennzeichnet, dass** die antigene Determinante mit dem C-Ende des Oligopeptids verbunden ist.

8. Zusammensetzung nach Anspruch 1, **dadurch gekennzeichnet, dass** die antigene Determinante ein Kohlenhydratepitop umfasst.

9. Zusammensetzung nach Anspruch 8, **dadurch gekennzeichnet, dass** das Kohlenhydratepitop von einem Bakterium stammt.

10. Zusammensetzung nach Anspruch 8, **dadurch gekennzeichnet, dass** das Kohlenhydratepitop von einem Virus stammt.

11. Zusammensetzung nach Anspruch 8, **dadurch gekennzeichnet, dass** das Kohlenhydratepitop von einer Krebszelle stammt.

12. Zusammensetzung nach Anspruch 8, **dadurch gekennzeichnet, dass** das Kohlenhydratepitop von einem Pilz stammt.

13. Zusammensetzung nach Anspruch 8, **dadurch gekennzeichnet, dass** das Kohlenhydratepitop von einem Parasiten stammt.

14. Zusammensetzung nach Anspruch 1, **dadurch gekennzeichnet, dass** das PADRE-Peptid außerdem kovalent an einen Lipidanteil gebunden ist.

15. Zusammensetzung nach Anspruch 14, **dadurch gekennzeichnet, dass** der Lipidanteil Palmitinsäure umfasst.

16. Zusammensetzung nach Anspruch 15, **dadurch gekennzeichnet, dass** der Lipidanteil PAM₂K ist, wo K ein Lysinrest und PAM ein Palmitinsäurerest ist.

17. Zusammensetzung nach Anspruch 14, **dadurch gekennzeichnet, dass** der Lipidanteil mit dem N-Ende des PADRE-Peptids verbunden ist

18. Zusammensetzung nach Anspruch 1, **dadurch gekennzeichnet, dass** das PADRE-Peptid die Formel R₁-R₂-R₃-R₄-R₅ vom N-Ende zum C-Ende aufweist, wo
R₁ aus mindestens zwei Resten besteht;
R₂ aus der Gruppe ausgewählt wird, die aus einem Cyclohexylalaninrest, einem Tyrosinrest, einem Phenylalaninrest und einer konservativen Substitution dafür besteht;
R₃ 3 bis 5 Reste umfasst;
R₄ aus der Gruppe ausgewählt wird, die aus Threonin-Leucin-Lysin, Lysin-Threonin und Tryptophan-Threonin-Leucin-Lysin und konservativen Substitutionen dafür besteht; und
R₅ aus mindestens zwei Resten besteht.

19. Zusammensetzung nach Anspruch 18, **dadurch gekennzeichnet, dass** jede Aminosäure von R₃ für sich aus der aus Alanin, Isoleucin, Serin und Valin bestehenden Gruppe ausgewählt wird.

20. Zusammensetzung nach Anspruch 18, **dadurch gekennzeichnet, dass** jede Aminosäure von R₅ für sich aus der aus Alanin, Serin und Valin bestehenden Gruppe ausgewählt wird.

21. Zusammensetzung nach Anspruch 18, **dadurch gekennzeichnet, dass**
R₁ D-Alanin, gefolgt von einen L-Alanin oder L-Lysin ist;
R₂ Cyclohexylalanin or Phenylalanin ist;
jeder Rest von R₃ aus der aus L-Alanin, Isoleucin und Valin bestehenden Gruppe ausgewählt wird:
R₅ 2 oder 4 L-Alanine, gefolgt von D-Alanin ist.

22. Zusammensetzung nach Anspruch 18, **dadurch gekennzeichnet, dass**
R₁ L-Alanin, gefolgt von einen L-Alanin oder L-Lysin ist;
R₂ Cyclohexylalanin oder Phenylalanin ist;
jeder Rest von R₃ aus der aus L-Alanin, Isoleucin und Valin bestehenden Gruppe ausgewählt wird; und
R₅ 2 oder 4 L-Alanine, gefolgt von L-Alanin ist.

23. Zusammensetzung nach Anspruch 18, **dadurch gekennzeichnet, dass** das PADRE-Peptid aus der aus aAXAAAKTAAAAa, aAXAAAATLKAAa, aAXVAAATLKAAa, aAXVAAATLKAAa, aAXIAAATLKAAa, aKXVAAWTLKAAa und aKFVAAWTLKAAa bestehenden Gruppe ausgewählt wird, wo a d-Alanin, A L-Alanin, X Cyclohexylalanin, K Lysin, T Threonin, L Leucin, V Valin, I Isoleucin, W Tryptophan und F Phenylalanin ist.

24. Zusammensetzung nach Anspruch 23, **dadurch gekennzeichnet, dass** das PADRE-Peptid aKXVAAWTLKAAa ist.

25. Zusammensetzung nach Anspruch 18, **dadurch gekennzeichnet, dass** das PADRE-Peptid aus der Gruppe ausgewählt wird, die aus AAXAAAKTAAAAA, AAXAAAATLKAAA, AAXVAAATLKAAA, AAXVAAATLKAAA, AAXIAAATLKAAA, AKXAAWTLKAAA und AKFVAAWTLKAAA besteht, wo A L-Alanin, X Cydohexylalanin, K Lysin, T Threonin, L Leucin, V Valin, I Isoleucin, W Tryptophan und F Phenylalanin ist.

26. Zusammensetzung nach Anspruch 18, **dadurch gekennzeichnet, dass** das PADRE-Peptid aus der Gruppe ausgewählt wird, die aus AAXAAAKTAAAAa, AAXAAAATLKAAa, AAXVAAATLKAAa, AAXVAAATLKAAa, AAXIAAATLKAAa, AKXVAAWTLKAAa und AKFVAAWTLKAAa besteht, wo a D-Alanin, A L-Alanin, X Cyclohexylalanin, K Lysin, T Threonin, L Leucin, V Valin, I Isoleucin, W Tryptophan und F Phenylalanin ist

27. Zusammensetzung nach Anspruch 18, **dadurch gekennzeichnet, dass** das PADRE-Peptid aus der Gruppe ausgewählt wird, die aus aAXAAAKTAAAAA, aAXAAAATLKAAA, aAXVAAATLKAAA, aAXVAAATLKAAA, aAXIAAATLKAAA, aKXVAAWTLKAAA und aKFVAAWTLKAAA besteht, wo a D-Alanin, A L-Alanin, X Cyclohexylalanin, K Lysin, T Threonin, L Leucin, V Valin, I Isoleucin, W Tryptophan und F Phenylalanin ist.

28. Pharmazeutische Zusammensetzung, **dadurch gekennzeichnet, dass** sie einen pharmazeutisch akzeptablen Carrier, eine nicht proteinische antigene Determinante und ein PADRE-Oligopeptid mit weniger als 50 Resten umfasst.

29. Zusammensetzung nach Anspruch 28, **dadurch gekennzeichnet, dass** sie außerdem ein Adjuvans umfasst.

30. Zusammensetzung nach Anspruch 29, **dadurch gekennzeichnet, dass** das Adjuvans das komplette Freundsche Adjuvans oder das inkomplette Freundsche Adjuvans ist.

31. Zusammensetzung nach Anspruch 28, die in einem Verfahren zum Auslösen einer humoralen Immunantwort genutzt wird, **dadurch gekennzeichnet, dass** die Zusammensetzung in ein Säugetier eingebracht wird.

32. Zusammensetzung nach Anspruch 31, **dadurch gekennzeichnet, dass** sie auf parenteralem Wege eingebracht wird.

33. Zusammensetzung nach Anspruch 31, **dadurch gekennzeichnet, dass** die Immunantwort prophylaktischer Art ist.

34. Zusammensetzung nach Anspruch 31, **dadurch gekennzeichnet, dass** die Immunantwort therapeutischer Art ist.

35. Zusammensetzung nach Anspruch 31, **dadurch gekennzeichnet, dass** die Immunantwort eine gegen die antigene Determinante des Anspruchs 1 gerichtete IgG Reaktion umfasst.

36. Zusammensetzung nach Anspruch 1, **dadurch gekennzeichnet, dass** die Zusammensetzung aus einem PADRE-Oligonukleotid mit weniger als etwa 50 Resten und der antigenen Determinante besteht.

## Revendications

1. Composition pour déclencher une réponse immunitaire contre un déterminant antigénique non protéique, la composition comprenant un oligopeptide PADRE de moins d'environ 50 résidus et le déterminant antigénique.

2. Composition selon la revendication 1, dans laquelle l'oligopeptide et le déterminant antigénique sont fixés l'un à l'autre.

3. Composition selon la revendication 2, dans laquelle l'oligopeptide et le déterminant antigénique sont liés par liaison covalente.

4. Composition selon la revendication 3, dans laquelle le déterminant antigénique est lié par liaison covalente au peptide PADRE par l'intermédiaire d'un séquence de liaison.

5. Composition selon la revendication 4, dans laquelle la séquence de liaison comprend un résidu cystéine.

6. Composition selon la revendication 4, dans laquelle le segment de liaison se compose d'un résidu acide aminocaproïque et d'un résidu cystéine.

7. Composition selon la revendication 1, dans laquelle le déterminant antigénique est lié à l'extrémité C terminale de l'oligopeptide.

8. Composition selon la revendication 1, dans laquelle le déterminant antigénique comprend un épitope glucidique.

9. Composition selon la revendication 8, dans laquelle l'épitope glucidique est dérivé d'une bactérie.

10. Composition selon la revendication 8, dans laquelle l'épitope glucidique est dérivé d'un virus.

11. Composition selon la revendication 8, dans laquelle l'épitope glucidique est dérivé d'une cellule cancéreuse.

12. Composition selon la revendication 8, dans laquelle l'épitope glucidique est dérivé d'un champignon.

13. Composition selon la revendication 8, dans laquelle l'épitope glucidique est dérivé d'un parasite.

14. Composition selon la revendication 1, dans laquelle le peptide PADRE est par ailleurs lié par liaison covalente à un groupement lipidique.

15. Composition selon la revendication 14, dans laquelle le groupement lipidique comprend l'acide palmitique.

16. Composition selon la revendication 15, dans laquelle le groupement lipidique est PAM₂K, où K est un résidu lysine et PAM est un résidu acide palmitique.

17. Composition selon la revendication 14, dans laquelle le groupement lipidique est lié à l'extrémité N du peptide PADRE.

18. Composition selon la revendication 1, dans laquelle le peptide PADRE a pour formule R₁-R₂-R₃-R₄-R₅, en allant de l'extrémité N terminale à l'extrémité C terminale, dans laquelle :
R₁ se compose d'au moins 2 résidus ;
R₂ est choisi dans le groupe constitué par un résidu cyclohexylalanine, un résidu tyrosine, un résidu phénylalanine et leurs substitutions conservatoires ;
R₃ est 3 à 5 résidus ;
R₄ est choisi dans le groupe constitué par les groupements thréonine-leucine-lysine, lysine-thréonine et tryptophane-thréonine-leucine-lysine, et leurs substitutions conservatoires ; et
R₅ se compose d'au moins 2 résidus.

19. Composition selon la revendication 18, dans laquelle chaque acide aminé de R₃ est choisi indépendamment dans le groupe constitué par les acides aminés alanine, isoleucine, sérine et valine.

20. Composition selon la revendication 18, dans laquelle chaque acide aminé de R₅ est choisi indépendamment dans le groupe constitué par les acides aminés alanine, sérine et valine.

21. Composition selon la revendication 18, dans laquelle:
R₁ est un résidu D-alanine suivi d'un résidu L-alanine ou L-lysine;
R₂ est un résidu cyclohexylalanine ou phénylalanine;
chaque résidu de R₃ est choisi dans le groupe constitué par les résidus L-alanine, isoleucine et valine; et
R₅ est constitué de 2 ou 4 résidus L-alanine suivis d'un résidu D-alanine.

22. Composition selon la revendication 18, dans laquelle :
R₁ est un résidu L-alanine suivi d'un résidu L-alanine ou L-lysine;
R₂ est un résidu cyclohexylalanine ou phénylalanine;
chaque résidu de R₃ est choisi dans le groupe constitué par les résidus L-alanine, isoleucine et valine; et
R₅ est constitué de 2 ou 4 résidus L-alanine suivis d'un résidu L-alanine.

23. Composition selon la revendication 18, dans laquelle le peptide PADRE est choisi dans le groupe constitué par aAXAAAKTAAAAa, aAXAAAATLKAAa, aAXVAAATLKAAa, aAXVAAATLKAAa, aAXIAAATLKAAa, aKXVAAWTLKAAa et aKFVAAWTLKAAa, où a est un résidu d-alanine, A est un résidu L-alanine, X est un résidu cyclohexylalanine, K est un résidu lysine, T est un résidu thréonine, L est un résidu leucine, V est un résidu valine, I est un résidu isoleucine, W est un résidu tryptophane, et F est un résidu phénylalanine.

24. Composition selon la revendication 23, dans laquelle le peptide PADRE est aKXVAAWTLKAAa.

25. Composition selon la revendication 18, dans laquelle le peptide PADRE est choisi dans le groupe constitué par AAXAAAKTAAAAA, AAXAAAATLKAAA, AAXVAAATLKAAA, AAXVAAATLKAAA, AAXIAAATLKAAA, AKXVAAWTLKAAA et AKFVAAWTLKAAA, où A est un résidu L-alanine, X est un résidu cyclohexylalanine, K est un résidu lysine, T est un résidu thréonine, L est un résidu leucine, V est un résidu valine, I est un résidu isoleucine, W est un résidu tryptophane et F est un résidu phénylalanine.

26. Composition selon la revendication 18, dans laquelle le peptide PADRE est choisi dans le groupe constitué par AAXAAAKTAAAAa, AAXAAAATLKAAa, AAXVAAATLKAAa, AAXVAAATLKAAa, AAXIAAATLKAAa, AKXVAAWTLKAAa et AKFVAAWTLKAAa, où a est un résidu D-alanine, A est un résidu L-alanine, X est un résidu cyclohexylalanine, K est un résidu lysine, T est un résidu thréonine, L est un résidu leucine, V est un résidu valine, I est un résidu isoleucine, W est un résidu tryptophane et F est un résidu phénylalanine.

27. Composition selon la revendication 18, dans laquelle le peptide PADRE est choisi dans le groupe constitué par aAXAAAKTAAAAA, aAXAAAATLKAAA, aAXVAAATLKAAA, aAXVAAATLKAAA, aAXIAAATLKAAA, aKXVAAWTLKAAA et aKFVAAWTLKAAA, où a est un résidu D-alanine, A est un résidu L-alanine, X est un résidu cyclohexylalanine, K est un résidu lysine, T est un résidu thréonine, L est un résidu leucine, V est un résidu valine, I est un résidu isoleucine, W est un résidu tryptophane et F est un résidu phénylalanine.

28. Composition pharmaceutique comprenant un véhicule pharmaceutiquement acceptable, un déterminant antigénique non protéique et un oligopeptide PADRE de moins de 50 résidus.

29. Composition selon la revendication 28, comprenant en outre un adjuvant.

30. Composition selon la revendication 29, dans laquelle l'adjuvant est l'adjuvant complet de Freund ou l'adjuvant incomplet de Freund.

31. Composition selon la revendication 28 à utiliser dans un procédé pour déclencher une réaction immunitaire humorale comprenant l'introduction de la composition dans un mammifère.

32. Composition selon la revendication 31, dans laquelle l'introduction est parentérale.

33. Composition selon la revendication 31, dans laquelle la réponse immunitaire est prophylactique.

34. Composition selon la revendication 31, dans laquelle la réponse immunitaire est thérapeutique.

35. Composition selon la revendication 31, dans laquelle la réponse immunitaire comprend une réponse IgG dirigée contre le déterminant antigénique de la revendication 1.

36. Composition selon la revendication 1, dans laquelle la composition se compose d'un oligonucléotide PADRE de moins d'environ 50 résidus et du déterminant antigénique.
